# EUROPEAN PATENT APPLICATION

(11) **EP 1 250 934 A1**
(43) Date of publication of application: **23.10.2002**
(21) Application number: 00964725.6
(22) Date of filing: 06.10.2000
(51) Int. Cl.: A61K 45/00, A61K 35/74, A61K 35/84, A61K 39/04, A61K 31/7016, A61K 31/702, A61P 43/00, A61P 35/00, A23L 1/212, A23L 1/28

(54) **ANTICANCER COMPOSITIONS**

(30) Priority: 28.01.2000 JP 2000024716; 17.03.2000 JP 2000075513; 24.03.2000 JP 2000128616; 28.04.2000 JP 2000131375
(71) Applicant: Orient Cancer Therapy Co. Ltd, Mitaka-shi, Tokyo 181-0015 (JP)
(72) Inventor: YAGITA, Akikuni, Mitaka-shi, Tokyo 181-0015 (JP)
(74) Representative: Forstmeyer, Dietmar, Dr. rer. nat., Dipl.-Chem.
(86) International application number: JP0006992
(87) International publication number: WO01054724

(57) **Abstract**

The present invention provides a novel means of immunological therapy for cancer. More particularly, it provides a therapeutic agent for cancer which is administered by using inducing ability for IL-12 production and NKT cell activating ability as indexes or a therapeutic agent for cancer or a supplementary food preparation for oral administration containing a substance having an activating ability of NKT cells which is a supplementary food preparation by oral administration given with an expectation of anticancer effect. The present invention further provides a mixed composition containing at least two of a processed product of mycelia of *Schizophyllum commune* or a saccharide derived from cultured filtrate of mycelia of *Schizophyllum commune* and processed product of mycelia of *Cortinellus shiitake* and a processed product of *Fomos glaucotus* as a compounded substance at least maintaining an inducing ability of IL-12 production and an activating ability of NKT cells. The present invention furthermore provides a novel preparation of IL-12 production inducer and a novel NKT cell activator.

## Description

### Background of the Invention

### 1. Field of the Invention

The present invention relates to a therapeutic agent for cancer paying attention to induction of production of interleukin-12 (hereinafter, may be sometimes referred to as "IL-12") and activation of natural killer T cell (hereinafter, may be sometime referred to as "NKT cell") and also relates to a supplementary food preparation for health by oral administration with an expectation of anticancer effect by paying attention to induction of production of IL-12 and activation of NKT cell. Thus, it relates to a therapeutic agent for cancer using an ability of induction of production of IL-12 and an ability of activation of NKT cell as indexes and also to a supplementary food preparation for health by oral administration which is taken with an expectation for anticancer effect.

### 2. Description of the Related Art

In screening the substances which are useful for prevention or therapy of cancer, their direct action to cancer cells has been considered to be important. On the other hand, usefulness of immunopotentiators has been noted already but all of compounds which have been prepared as immunopotentiators have weak anticancer effect and, by means of an immunotherapy or even by means of a joint therapy with chemotherapy, a sufficient therapeutic effect for cancer has not been achieved.

Dr. Yagita who is the inventor for the present invention previously paid his attention to the usefulness of a substance which induced IL-12 *in vivo* as an epoch-making means in cancer therapy. He found that AHCC which is a processed product of mycelia of *Cortinellus shiitake* had such a function and established a therapeutic method for cancer which might be said to be a novel immunotherapy. There has been a fact that, up to now, although IL-12 *per se* has an anticancer effect, it results in a side effect when IL-12 is directly administered *in vivo* whereby patients are not endurable to the therapy. In view of the above, IL-12 *per se* has not been able to be used as an anticancer agent. However, a preparation containing AHCC reported by Dr. Yagita achieved significant curing and macrobiotic effects in the therapy of cancer. Thus, he succeeded in curing cancer by administration of AHCC in a dose which is effective for induction of IL-12 *in vivo* (Japanese Patent Laid-Open No. 10/139670).

IL-12 has an action of potentiating the production of interferon γ (hereinafter, may be sometimes referred to as "IFNγ") and action for activation and potentiation of natural killer cells (hereinafter, may be sometimes referred to as "NK cells"), lymphokine activated killer cells and killer T cells having a role of cellular immune *in vivo.* IFNγ is a cytokine which induces the immune response of living body to a state where T helper 1 cells (hereinafter, may be sometimes referred to as "Th1" act (the state where NKT cells and killer T cells are apt to achieve the effect or, in other words, the state where interleukin-2 and IL-12 are produced in large quantities). Killer T cells and LAK cells have been known as the cells participating in cancer immune. NK cells have been also reported to participate in anticancer action of living body. However, Dr. Yagita has proved that activity of NK cells do not correlate to the clinical anticancer effect but, rather, induced production amount of IL-12 and NK activity are in an entirely reversed correlation and, therefore, it is concluded that NK cells do not participate in the anticancer action in human being.

It has been established by Dr. Yagita at present that the substances having an ability of inducing the production of IL-12 have a possibility of becoming as promising anticancer substances and that has been confirmed by the present invention as well.

However, in some patients suffering from cancer, production of IL-12 is not sufficiently induced even by administration of AHCC whereby a therapeutic effect is not achieved and, even if production of IL-12 is induced, a therapeutic effect is not achieved. Under such circumstances, there has been a further demand for the development of a novel therapeutic agent for cancer which acts by a mechanism being different from the anticancer effect of AHCC.

It has been known already that, in the action mechanism of cancer immune, amount of cytokine produced or induced *in vivo* is an important factor and a method for the therapy of cancer by administration, induction or production of cytokine which is said to have an anticancer effect has been also attempted and carried out. In spite of the fact that the relations between cancer and immune and between cancer and cytokine have been made clear, however curing and macrobiotic effect for cancer has been accomplished in the patients of not more than only 50%.

In addition, NKT cell (Cui, J. et al., *Science*, 278, 1623, 1997) has been found recently as a cell participating in cancer immune. The NKT cell is one of the cells participating in immune system and has a strong cytokine-producing ability, particularly IFNγ-producing ability, a function of cytotoxicity mediated by Fas or perforin and et al. Therefore, it is expected that curing and macrobiotic effect to the patients suffering from cancer will become higher when the said cell is activated. Taniguchi, et al. found a specific glycolipid antigen recognized by Vα24Vβ11 which is a special T cell receptor in the NKT cell and reported that the said antigen is α-galactosylceramide. It was further proved that, in cancer-carrying mouse to which α-galactosylceramide was administered, NKT cell was activated and, although disappearance of cancer was not observed, metastasis of cancer was suppressed (Kawano, T., et al., *Proc. Natl. Acad. Sci., USA.,* 95: 5690, 1998).

Further, utilization of BCG live vaccine which is one of immunopotentiator as an anticancer agent in immunotherapy has been carried out within a very limited range. However, there has been no report for the relation between the BCG live vaccine and activation of NKT cell and for the relation between the vaccine and the amount of IL-12. With regard to BCG CWS (BCG cell wall skeleton) which is a membrane component derived from BCG, there was investigated the possibility of activation by BCG CWS of NKT cells but a sufficient effect was not achieved and investigation thereafter was given up.

### Disclosure of Invention

A problem which is to be solved by the present invention is to provide an effective therapeutic agent for cancer on the basis of information of cancer immune cascade and a method of administering the same and to provide a more effective means for the therapy of cancer.

In order to solve the above-mentioned problem, the present inventor has carried out repeated investigations on a cancer immune cascade in prevention or therapy of cancer and found the presence of two cascades carrying the cancer immune, i.e. a cascade in which the induced and produced IL-12 is participated and another cascade in which the activated NKT cell is participated. It has been also found that the fact of making the rate of T helper 1 cell (Th1) carrying the immune function to T helper 2 cell (Th2) high is an important index for judging the effect of a therapeutic agent for cancer. On the basis of such a finding, it has been found that, when the effect of therapeutic agent for cancer is judged in the therapy of cancer using the IL-12 production inducing ability and the NKT cell activating ability as indexes and its type, dose and administration route are appropriately selected, the therapeutic effect to cancer is significantly improved. The present inventor has further found that the live vaccine of BCG and the substance derived from mycelia of mushroom activate the NKT cells *in vivo* in the patients suffering from cancer whereupon the present invention has been achieved.

### Summary of the Invention

Thus, the present invention comprises the characteristic features which will be mentioned as follows.
1. A therapeutic agent for cancer where an inducing ability for the production of interleukin 12 (IL-12) and an activating ability for NKT cells are used as indexes.
2. The therapeutic agent for cancer according to the above 1, wherein the cell ratio of Th1/Th2 being about 7.0 or more is used as an index for the effect.
3. The therapeutic agent for cancer according to the above 1, wherein the agent contains a substance having an activating ability for NKT cells which is administered to a patient where the amount of IL-12 produced *in vivo* is low even by administration of an inducing agent for the production of IL-12 or to a patient where the therapeutic effect for cancer is not achieved even when the IL-12 value is high.
4. The therapeutic agent for cancer according to any of the above 1 to 3, wherein it is a supplementary food preparation for health by oral administration.
5. A composition which activates the NKT cells where at least one selected from live cell, attenuated live cell and a thing derived from mycelia of mushroom is an active ingredient.
6. The composition according to the above 5, wherein the substance having a function of activating the NKT cells is BCG live vaccine or a thing derived therefrom.
7. The composition according to the above 5, wherein the thing derived from mycelia of mushroom having a function of activating the NKT cells is selected from a processed product of mycelia of *Schizophyllum commune,* a polysaccharide derived from cultured filtrate of mycelia of *Schizophyllum commune,* a processed product of mycelia of *Coriolus versicolor* and a processed product of mycelia of *Cortinellus shiitake.*
8. The composition according to the above 7, wherein the processed product of mycelia of *Schizophyllum commune* is SCP, the polysaccharide derived from culture filtrate of mycelia of *Schizophyllum commune* is SPG, the processed product of mycelia of *Coriolus versicolor* is PSK (Krestin®) and the processed product of mycelia of *Cortinellus shiitake* is AHCC and LEM.
9. The composition according to the above 5, wherein the substance having a function of activating the NKT cells is a saccharide having α-1,3 and/or α-1,4 glucoside bond structure(s).
10. The composition according to the above 9, wherein the saccharide is a polysaccharide and/or an oligosaccharide.
11. A composition which is characterized in compounding at least a substance having an inducing ability for the production of IL-12 and a substance having a function of activating the NKT cells.
12. The composition according to the above 11, wherein at least one component selected from a processed product of mycelia of *Schizophyllum commune,* a polysaccharide derived from cultured filtrate of mycelia of *Schizophyllum commune,* a processed product of mycelia of *Cortinellus shiitake,* a processed product of mycelia of *Fomos glaucotus* and a processed product of mycelia of *Coriolus versicolor* as the substance having an inducing ability of production of IL-12 and at least one component selected from a product derived from BCG live vaccine, a processed product of mycelia of *Coriolus versicolor,* a processed product of mycelia of *Schizophyllum commune,* a polysaccharide derived from cultured filtrate of mycelia of *Schizophyllum commune* and a polysaccharide having α-1,3 and/or α-1,4 glucoside bond structure(s) as the substance having an activating ability to NKT cells are compounded.
13. The composition according to the above 12, wherein the processed product of mycelia of *Schizophyllum commune* is SCP, the polysaccharide derived from cultured filtrate of mycelia of *Schizophyllum commune* is SPG, the processed product of mycelia of *Cortinellus shiitake* is AHCC and LEM, the processed product of mycelia of *Fomos glaucotus* is SM and MAK and the processed product of mycelia of *Coriolus versicolor* is PSK (Krestin®).
14. The composition according to the above 12, wherein three kinds of substances - the processed product of mycelia of *Schizophyllum commune* or the polysaccharide derived from cultured filtrate of mycelia of *Schizophyllum commune*, the processed product of mycelia of *Cortinellus shiitake* and the processed product of mycelia of *Fomos glaucotus -* are compounded.
15. The composition according to the above 14, wherein 30∼50 parts by weight of the processed product of mycelia of *Schizophyllum commune* or the polysaccharide derived from cultured filtrate of mycelia of *Schizophyllum commune,* 30∼50 parts by weight of the processed product of mycelia of *Cortinellus shiitake* and 10∼30 parts by weight of the processed product of mycelia of *Fomos glaucotus* are compounded.
16. The composition according to the above 14 or 15, wherein the processed product of mycelia of *Schizophyllum commune* is SCP, the polysaccharide derived from cultured filtrate of mycelia of *Schizophyllum commune* is SPG, the processed product of mycelia of *Cortinellus shiitake* is AHCC or LEM, the processed product of mycelia of *Fomos glaucotus* is SM or MAK and the processed product of mycelia of *Coriolus versicolor* is PSK (Krestin®).
17. A composition for inducing the production of IL-12 in which at least one which is selected from a processed product of mycelia of *reishi (*bracket fungus of the genus *Fomos), a* processed product of mycelia of *Ganoderma boninense* and a processed product of mycelia of *Coriolus versicolor* is contained as an active ingredient.
18. The composition for inducing the production of IL-12 according to the above 17, wherein the processed product of *reishi (*bracket fungus of the genus *Fomos)* is MAK, the processed product of mycelia of *Ganoderma boninense* is SM and the processed product of mycelia of *Coriolus versicolor* is PSK (Krestin®).
19. The composition for inducing the production of IL-12 according to the above 17 or 18, wherein it is administered to a patient suffering from progressive or terminal cancer.
20. A composition for inducing the production of IL-12 to be administered to a patient suffering from primary cancer in which at least one which is selected from a processed product of mycelia of *Schizophyllum commune,* a polysaccharide derived from cultured filtrate of mycelia of *Schizophyllum commune* and a processed product of mycelia of *Cortinellus shiitake*.
21. The composition for inducing the production of IL-12 according to the above 20, wherein the processed product of mycelia of *Schizophyllum commune* is SCP, the polysaccharide derived from cultured filtrate of mycelia of *Schizophyllum commune* is SPG and a processed product of mycelia of *Cortinellus shiitake* is AHCC and LEM.
22. A composition for inducing the production of IL-12 in which a processed product of mycelia having an ability of inducing the production of IL-12 in a patient suffering from progressive or terminal cancer and a processed product of mycelia having an ability of inducing the production of IL-12 in a patient suffering from primary cancer are compounded.
23. The composition for inducing the production of IL-12 according to the above 22, wherein at least three selected from a processed product of mycelia of *Schizophyllum commune* or a polysaccharide derived from cultured filtrate of mycelia of *Schizophyllum commune,* a processed product of mycelia of *Cortinellus shiitake,* a processed product of mycelia of *Fomos glaucotus* and a processed product of mycelia of *Coriolus* *versicolor* are compounded as active ingredients.
24. The composition for inducing the production of IL-12 according to the above 23, wherein the processed product of mycelia of *Schizophyllum commune* is SCP, the polysaccharide derived from cultured filtrate of mycelia of *Schizophyllum commune* is SPG, the processed product of mycelia of *Cortinellus shiitake* is AHCC and LEM, the processed product of mycelia of *Fomos glaucotus* is SM and MAK and the processed product of mycelia of *Coriolus versicolor* is PSK (Krestin®).
25. A therapeutic agent for cancer containing any of the compositions mentioned in the above 5 to 24, characterized in that, the agent is in a form of being administered *per os.*
26. A supplementary food preparation for health by oral administration containing any of the compositions mentioned in the above 5 to 24.
27. A commercial medium carrying any of the contents mentioned in the above 1 to 26.
28. A commercial method utilizing any of the contents mentioned in the above 1 to 26.
29. In a method for testing the activation of NKT cells, a method for testing the activation of NKT cells which is characterized in that the cell surface antigens CD161 and CD3 are measured.
30. In a system where immunotherapy of cancer is carried out under tie-up and cooperation of an organization for testing the immune function, an organization for evaluating the immune function and an organization for therapy and/or formulation (a medical organization), a novel cancer immunotherapy system comprising
   (1) when blood of a patient suffering from cancer is collected at the medical organization and sent to the organization for testing the immune function,
   (2) measurement of at least IL-12, NKT activity and IFNγ is carried at out the said organization for testing,
   (3) result of the measurement is sent to the tied-up organization for evaluating the immune function and an evaluation is carried out in the said organization on the basis of the result of the examination,
   (4) whereby selection of the adaptability with the formulation of the above 1 to 26 is carried out,
   (5) the said information is transmitted to the organization for therapy and/or formulation and
   (6) a cancer immunotherapy is carried out on the basis of the said transmition.
31. A method for the cancer immunotherapy by any of the formulations of the above 1 to 26.

### Brief Description of the Drawings

Fig. 1 is a drawing which shows the activation of NKT cells after administration of BCG live vaccine for four times. The result is a mean value of 19 cases of patients to whom the BCG live vaccine was administered.

Fig. 2 is a drawing which shows condition of a reversed correlation between produced amount of IL-12 and activation of NKT cells in a patient suffering from cancer to whom the composition containing the IL-12 production inducer and the NKT cell activator according to the present invention was orally administered.

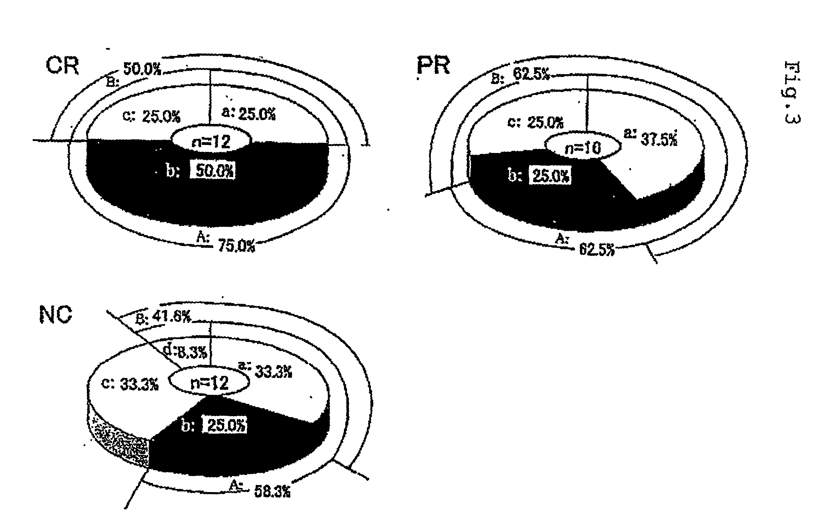

Fig. 3 is a drawing which shows the rate of the patients where NKT cells and/or IL-12 productivity were/was activated in the patients suffering from cancer to whom the composition containing the IL-12 production inducer and the NKT cell activator according to the present invention was orally administered is given for each of completely recovered patients (CR), partially recovered patients (PR) and non-reactive patients (NC). In the drawing, "a" shows the rate of the patients suffering from cancer where NKT cells were activated and productivity of IL-12 was positive, "b" shows the rate of the patients where only NKT cell activation was noted, "c" shows the rate of the patients where only IL-12 productivity was positive and "d" shows the rate of the patients where both were inactivated. In the drawing, "A" shows the rate of the patients where the rate of the NKT cells in mono nuclear cells was 10% or more while "B" shows the rate of the patients where the IL-12 production amount was 7.8 pg/ml or more.

Fig. 4 is a drawing which shows that there is a correlation between CD3(+)CD161 (+) cells and CD3(+)CD161(+)CD56(+) cells.

### Detailed Description of the Preferred Embodiments

The present invention will now be illustrated in detail as hereunder.

With regard to the patients suffering from cancer to who a composition containing an IL-12 production inducer and an NKT cell activator of the present invention was orally administered , the present inventor measured the IL-12 productivity and NKT cells and analyzed and, as a result, it has been found that a cancer therapy by induction of production of IL-12 and a cancer therapy by activation of NKT cells are the systems which are independent each other and accordingly that, in order to achieve a high therapeutic effect in the therapy of cancer, it is necessary to achieve both induction of production of IL-12 and activation of NKT cells.

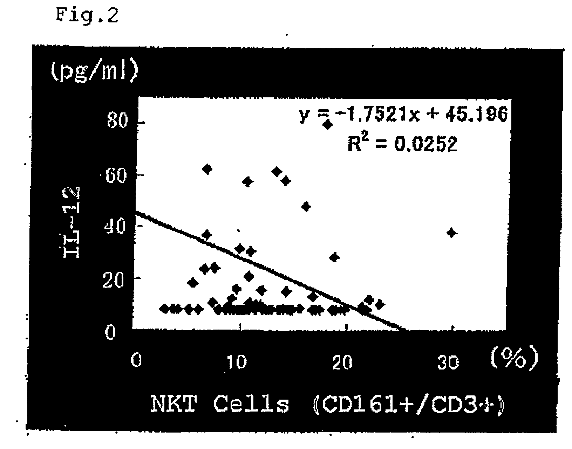

As shown in Fig. 2, IL-12 productivity and NKT cell activity clear showed a reverse correlation in all of 79 cases of patients suffering from cancer after about three months of oral administration of a composition containing the IL-12 productivity inducer and NKT cell activator according to the present invention. The result suggests the fact that a system concerning the induction of IL-12 production and a system concerning the activation of NKT cells function independently each other.

Fig. 3 shows that, in the patients suffering from cancer who were treated as same as above, the rate of the patients where IL-12 productivity and/or NKT cells are/is activated is shown for each of the completely recovered (hereinafter, that may be sometimes abbreviated as CR) patients (4 weeks or longer have elapsed since the disappearance of cancer), the partially recovered (hereinafter, that may be sometimes abbreviated as PR) patients (50% or more cancer became smaller) and non-reactive (hereinafter, that may be sometimes abbreviated as NC) patients (growth of cancer was suppressed to 50% or less or suppression of 25% or less growth was noted). With regard to the productivity of IL-12, the case where the productivity was 7.8 pg/ml (a quantitative limit) or more was defined as positive. In the CR cases, 6 cases (50%) among 12 cases were judged to be positive for the IL-12 productivity. One half among them, i.e. 3 cases (25%), activation of NKT cells was noted as well and, as a cellular immune system, it was found that both NKT cell system and killer T cell system activated by the production of IL-12 participated in the recovery of cancer. The cases believed to be completely recovered by participation of the NKT cells were 9 cases (75%). Among them, the cases believed to be completely recovered only by activation of the NKT cells were 6 cases (50%) and, in other 3 cases, both killer T cell system and NKT cell system participated as mentioned above. Among the PR cases, both IL-12 production and NKT cell were activated in 37.5% of the cases and both killer T cell system and NKT cell system participated therein. Among the PR cases, the cases where only NKT activation was noted or the cases where the participation of only killer T cell system was noted were 25% each.

In the present invention, the cell ratio of Th1/Th2 of the patients suffering from cancer was tested by a helper T (Th) cell system three color analytical test by means of a flow cytometry. Th1/Th2 means the rate of cells which produce IFNγ (Th1) to the cells which produce IL-4 (Th2) among the helper T cells having CD4 which is one of the cell surface antigens and is also expressed as "CD4 × IFNγ/IL-4". In the present invention, it has been confirmed that the treatment of the present invention comprising the means of induction of production of IL-12 and/or activation of NKT cells is effective for the therapy of cancer where the fact that Th1/Th2 of the patients suffering from cancer becomes at least about 7.0 or more is used as an index. In all of the cases where cancer became small by administration of the therapeutic agent or composition of the present invention, the cell ratio of Th1/Th2 showed a value of 7 or more. On the other hand, in the NR cases where growth of cancer was noted, the case where the said value became more than 7.0 was nil among the 12 cases. Accordingly, when the said value of the patient suffering from cancer is about 7 or less using the cell ratio of Th1/Th2 as an index, application of a treatment for making this value about 7 or more such as the use of the therapeutic agent or composition of the present invention is effective for the therapy of cancer.

The present invention provides a therapeutic agent for cancer which is administered where an ability of inducing the IL-12 production and an ability of activating the NKT cells are indexes and also provides a supplementary food preparation for oral administration which is administered with an expectation of anticancer effect. The present invention particularly provides a therapeutic agent for cancer containing a substance having an activating ability for NKT cells which is applied to a patient where the IL-12 value is low even when an inducer for IL-12 production is administered or where the therapeutic effect for cancer is not achieved even when the IL-12 value is high and also provides a supplementary food preparation for oral administration which is administered with an expectation of anticancer effect.

A therapeutic agent for cancer containing a substance of the present invention having an activating ability for NKT cells contains an effective amount of a substance having an activating ability for NKT cells and, preferably, administered *per os*.

A supplementary food preparation for health by oral administration which is administered with an expectation of anticancer effect containing a substance having an activating ability for NKT cells according to the present invention is a supplementary food preparation for health by oral administration where an anticancer effect can be expected as a result of administration.

The therapeutic agent according to the present invention is effective for the therapy of lung cancer, pulmonary adenomatosis, thymoma, thyroid cancer, bladder cancer, colon cancer, rectum cancer, cecum cancer, urinary tract cancer, breast cancer, uterine cervex cancer, brain tumor, cancer of the tongue, throat cancer, nasal cavity cancer, laryngeal cancer, gastric cancer, hepatoma, bile duct cancer, testicular cancer, ovarian cancer, cancer of uterine boby, malignant melanoma, liposarcoma, etc. although they are not limitative. Particularly, the agent is appropriately administered to a patient where IL-12 amount is low (such as not more than 7.8 pg/ml) even when an inducer of IL-12 production such as AHCC (K. K. Aminoup) is administered or to a patient where the said amount is high but no improvement is noted for the therapy of cancer. Further, a high therapeutic effect can be expected when it is applied to a patient where the amount of interferon γ (IFNγ) which is one of cytokines participating in cancer immune having an important action in a cancer immune cascade is low. Furthermore, the supplementary food preparation for health by oral administration according to the present invention may be administered to the patient as such with an expectation of an anticancer effect.

Measurement of the amount of IL-12 induced in a patient suffering from cancer is carried out for a culture liquid which is prepared in such a manner that a stimulating substance is added to a spherical blood mono nuclear cells which are separated and prepared from the blood of the said patient, and the resulted cells are incubated and subjected to a centrifugal separation to remove the sedimented cells. Density of the cells used for the incubation is from 0.5 × 10⁶ cells/ml to 1 × 10⁷ cells/ml or, preferably, 1 × 10⁶ cells/ml. With regard to a substance for stimulating the cells, phytohemagglutinin (PHA) which is a conventionally used mitogen is added to make its final concentration 0.1∼100 µg/ml or, preferably, 1∼20 µg/ml and then incubation is carried out. The substance which stimulates the cells is not limited to PHA but any substance may be used so far as it is able to produce an immunophysiologically active substance as a result of stimulation of the cells for achieving the object of the present invention and its examples are PMA (phorbol 12-myristate-13-acetate), PMA + ionomycin, LPS (lipopolysaccharide) and PWM (poke weed mitogen). Measurement of the amount of IL-12 may utilize the clinical and biochemical tests which have been known *per se* and there may be used a measurement kit by means of an enzyme-linked immunosorbent assay (ELISA) which is available from R&D Systems and MBL. In the present invention, an ability for inducing the IL-12 production means a function where the amount of IL-12 produced by stimulation of peripheral mono nuclear cells is potentiated to 7.8 pg/ml or more or, in case a substance is administered, it means a function where the IL-12 production amount after administration of the said substance is potentiated as compared with that before the administration.

Measurement of activation of NKT cells can be carried out by checking the increase in the NKT cell numbers by means of measuring the cell surface antigens which are specifically present on the cell surface of the NKT cells. To be more specific, cells which are CD3-positive cells and also CD161-positive cells are tested for mono nuclear cells in peripheral blood. Thus, CD3 and CD161 which are cell surface antigens of NKT cells are measured by a Two Color test using a flow cytometry by the use of monoclonal antibody. The fact that NKT cells are activated in the present invention means that the rate of the NKT cells in the monocytes is 10% or more. NKT cell activating ability means a function where the rate of the NKT cells is increased to an extent of 10% or more or a function where, in case a substance is administered, the rate of the NKT cells after the administration the substance is further potentiated than that before the said administration.

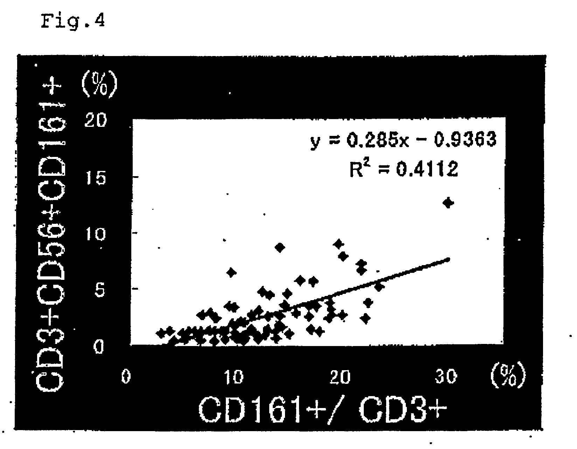

In the conventional means for measuring the NKT cell activation, it has been believed to be necessary that the three cell surface antigens which are CD3, CD161 and CD56 are measured. However, the present inventor has found for the first time that, in the detection of the NKT cells, the measured result for CD3 and CD161 is identical with that for CD3, CD161 and CD56 (Fig. 4). In the present invention, a method for the measurement of two cell surface antigens which are CD3 and CD161 is provided as a method for measuring the NKT cells. With regard to the method for the measurement of the cell surface antigens and to the reagents used therefor which are anti-CD3 antibody and anti-CD 161 antibody, the conventionally used method and reagents used for a method of measuring the three cell surface antigen may be used. As compared with the conventional method, the measuring method of the present invention is simple and convenient, cost-saving and useful.

The composition for inducing the production of IL-12 used in the present invention contains at least one of processed product of mycelia of *Cortinellus shiitake* such as AHCC and LEM (Noda Shokkin Kogyo K. K.), substances derived from mycelia of *Schizophyllum commune* such as SPG (Sizofiran; Kaken Pharmaceutical Co., Ltd.)(a polysaccharide obtained from culture filtrate of mycelia of *Schizophyllum commune)* and SCP (processed product of mycelia of *Schizophyllum commune* for oral administration; Yugen Kaisha Tozai Iyaku Kenkyusho), processed product of mycelia of *Fomos glaucotus* such as MAK (*reishi* or bracket fungus of the genus *Fomos)* (Noda Shokkin Kogyo K. K.) and SM (*Ganoderma boninense)* and processed product of *Coriolus* *versicolor* such as PSK (Krestin®) as an active ingredient.

Incidentally, in the present invention, the term reading "a substance derived from" means and includes all of polysaccharide, processed product of mycelia, etc. obtained from cultured filtrate of mushroom and it is used in the same meaning throughout the present invention.

The present inventor has found that a composition containing such substances as an active ingredient which induces IL-12 production shows a big difference in the ability for inducing the IL-12 production in each of the stages of progress of cancer. Thus, an agent containing at least one thing selected from a processed product of mycelia of *Fomos glaucotus* such as MAK and SM (*Ganoderma boninense*) and a processed product of mycelia of *Coriolus versicolor* such as PSK shows a sufficient inducing ability for IL-12 production even in a primary stage of cancer and, characteristically, it achieves the same or even stronger inducing ability for IL-12 production in the progressive terminal cancer as well. On the other hand, an agent containing at least one thing selected from a processed product of mycelia of *Cortinellus shiitake* such as AHCC and LEM, a processed product of mycelia of *Schizophyllum commune* such as SPG and SCP and polysaccharide derived from culture filtrate of mycelia of *Schizophyllum commune* as an active ingredient achieves a characteristic inducing ability for IL-12 production in primary stage of cancer but, with a progress of cancer, its inducing ability decreases.

Accordingly, in order to achieve the object of the present invention, it is necessary that those processed products of mycelia of mushrooms and compositions thereof are used after selection depending upon the state of progress of cancer. On the basis of such a finding, the present invention provides a composition for the induction of production of IL-12 containing at least one thing selected from a processed product of mycelia of *reishi* such as MAK, a processed product of mycelia of *Ganoderma boninense* such as SM and a processed product of mycelia of *Coriolus versicolor* such as PSK (Krestin®) as an active ingredient to be administered to a patient suffering from progressive or terminal cancer. It also provides a composition for the induction of production of IL-12 containing at least one thing selected from a processed product of mycelia of *Schizophyllum commune* such as SCP, polysaccharide derived from cultured filtrate of mycelia of *Schizophyllum commune* such as SPG and a processed product of mycelia of *Cortinellus shiitake* such as AHCC and LEM as an active ingredient to be administered to a patient suffering from primary cancer. Further, the composition for the induction of production of IL-12 according to the present invention may also be a composition for inducing the IL-12 production in which a processed product of mycelia of mushroom having an IL-12 production inducing ability for progressive or terminal cancer and a processed product of mycelia of mushroom having an IL-12 production inducing ability for primary cancer are compounded.

Dose of the composition for induction of production of IL-12 is around 1∼2,000 mg/kg body weight per day and the composition is appropriately administered *per os* during from 10 days to one year for one to several times a day. Needless to say, it is also possible to be parenterally administered by reducing the dose so that the compound is prepared into a quality durable for a parenteral administration.

In the composition having an activating ability for NKT cells according to the present invention, at least one thing which is selected from live cells, attenuated live cells and a thing derived from mycelia of mushroom is an active ingredient. One of the specific examples thereof is BCG live vaccine or a thing derived therefrom. With regard to the BCG live vaccine, that which is commercially available as a therapeutic agent for tuberculosis is used. When this is used as vaccine for tuberculosis, it is common to administer percutaneously and it has been known that immunological competence of living body is promoted by one or more administration(s). In the present invention, there is provided a composition which activates the NKT cells and contains BCG or, to be more specific, about 10∼500 mg or, more preferably, 40∼100 mg of living cells, amount of BCG. The said composition or a preparation containing the said composition is administered preferably *per os*. Dose and period for administration may be applied after testing the activation of the NKT cells in blood of a patient suffering from cancer and selecting the amount and the period for activating the NKT cells can be activated. An example for the administering method is that about 80 mg of living cell preparation are administered every 3∼10 days for one ∼ several months. As a result of oral administration of BCG live vaccine, fever of 38°C or higher, diarrhea and stomachache may be noted but side effect is rarely noted.

Another example of a substance having an activating ability for NKT cells is a substance derived from mycelia of mushroom and the effective ones are a substance derived from mycelia of *Schizophyllum commune* such as SPG (Sizofiran; Kaken Pharmaceutical Co., Ltd.) (a polysaccharide obtained from cultured filtrate of mycelia of *Schizophyllum commune*) and SCP (a preparation for oral administration of a processed product of mycelia of *Schizophyllum commune)* (Yugen Kaisha Tozai Iyaku Kenkyusho), a processed product of *Coriolus versicolor* such as PSK (Krestin®) and a processed product of mycelia of *Cortinellus shiitake* such as AHCC and LEM. SPG (Sizofiran; Kaken Pharmaceutical Co., Ltd.) which is a polysaccharide obtained from cultured filtrate of mycelia of *Schizophyllum commune* has been used as an anticancer agent against a partially limited cancer (Taito K. K.; Kaken Pharmaceutical Co., Ltd.). PSK (Krestin®) is in the same situation as well.

In the present invention, inducing ability for the production of IL-12 and activating ability for NKT cells *in vivo* are used as indexes whereby the usefulness of a processed product of mycelia of *Schizophyllum commune,* a polysaccharide derived from cultured filtrate of mycelia of *Schizophyllum commune,* a processed product of mycelia of *Coriolus versicolor* such as PSK (Krestin®) and a processed product of mycelia of *Cortinellus shiitake* such as AHCC and LEM has been rediscovered whereupon the present invention has been achieved. Thus, the present invention provides a composition which activates the NKT cells containing at least one thing selected from a polysaccharide derived from cultured filtrate of mycelia of *Schizophyllum commune* such as SPG, a processed product of mycelia of *Schizophyllum commune* such as SCP, processed product of mycelia of *Coriolus versicolor* such as PSK (Krestin®) and a processed product of mycelia of *Cortinellus shiitake* such as AHCC and LEM as an active ingredient.

On the basis of such a finding, the present inventor has found that a substance derived from mycelia of mushroom having an activating ability for NKT cells is a saccharide component having α-1,3 and/or α-1,4 glucoside bond structure or, particularly preferably, having at least α-1,3 glucoside bond structure. It has been also found that a substance having an activating ability for the NKT cells may be a composition of a thing derived from mycelia of mushroom containing the polysaccharide and/or 2∼10 oligosaccharides having such a structure. The present inventor has investigated the substances derived from mycelia of various mushrooms and NKT activating ability and investigated the relation between the component thereof and the NKT activating ability whereupon the presence of the above-mentioned saccharide structure is found to be an essential feature of the NKT cell activating ability of the thing derived from mycelia of mushroom. It has been further confirmed that β-1,3 and β-1,6 glucoside bond structures of mycelia also have an activating ability for NKT cells.

Dose of the composition which activates the NKT cells containing such a thing derived from mycelia of mushroom is around 1∼2,000 mg/kg per day during 10 days to one year for one to several times a month and, appropriately, it is administered *per os*. Needless to say, it is also possible to be parenterally administered by reducing the dose so that the compound is prepared into a quality durable for a parenteral administration.

With regard to a composition of the present invention activating the NKT cells, each of the above-mentioned processed products of mycelia of mushrooms having an activating ability for NKT cells or the things derived from mycelia of mushrooms may be used solely or they may be used jointly at the same time.

In the meanwhile, it has been found by the present inventor that, when BCG live vaccine is administered *per os*, although it shows an activating ability for NKT cells, an IL-12 producing ability is suppressed. Therefore, its use is to be limited to the case where the IL-12 productivity is low and, in addition, growth of cancer is unable to be suppressed.

The present invention also relates to a therapeutic agent for cancer where at least a substance having an IL-12 productivity inducing ability and a substance having NKT cell activating ability are compounded or to a supplementary food preparation for health by oral administration which is administered with an expectation of anticancer effect. The substance having an ability inducing the IL-12 productivity and the substance having an ability of the NKT cell activation are selected from the above-exemplified substances.

With regard to examples of such a compounded composition, at least two things selected from a processed product of mycelia of *Schizophyllum commune* such as SCP, a polysaccharide derived from cultured filtrate of mycelia of *Schizophyllum commune* such as SPG, a processed product of mycelia of *Cortinellus shiitake* such as AHCC and LEM and a processed product of mycelia of *Ganoderma boninense* such as MAK and SM are compounded. It has been widely known that most of them have been used already as immunopotentiators with an object of anticancer effect. However, in the present invention, the relation of such a combination with the IL-12 production inducing ability and the NKT cell activation has been found and, as a result of such a combination, a superiority (efficacy of 50% or more) which is far better than the anticancer effect (efficacy of 20%) of the common anticancer agents up to now has been established whereupon the present invention has been completed.

The optimum combination is a composition where the three things - a processed product of mycelia of *Schizophyllum commune* or a polysaccharide derived from cultured filtrate of mycelia of *Schizophyllum commune,* a processed product of mycelia of *Cortinellus shiitake* and a processed product of mycelia of *Fomos glaucotus -* are compounded and, to be more specific, it is a composition where 20∼60 parts by weight or, preferably, 30∼50 parts by weight of the processed product of mycelia of *Schizophyllum commune* or the polysaccharide derived from cultured filtrate of mycelia of *Schizophyllum commune*, 20∼60 parts by weight or, preferably, 30∼50 parts by weight of the processed product of mycelia of *Cortinellus shiitake* and 5∼40 parts by weight or, preferably, 10∼30 parts by weight of the processed product of mycelia of *Fomos glaucotus* are compounded. Such a compounded composition is useful as a therapeutic agent for cancer or as a supplementary food preparation for health by oral administration with an expectation of anticancer effect.

Since the relation between the state of progress of cancer and the IL-12 productivity inducing ability of various processed products of mycelia of mushrooms has been found in the present invention, additional usefulness of the above-mentioned combination has been confirmed.

Dose of the compounded composition of the present invention by oral administration is usually around 1∼2,000 mg/kg body weight per day for adults. Use and dose can be adjusted depending upon the induced IL-12 product amount and/or degree of activation of NKT cells. Term for the administration is from 10 days to one year and frequency of the administration is from one to several times a month.

A polysaccharide obtained from cultured filtrate of mycelia of *Schizophyllum commune* has been commercially available as SPG (Sizofiran) from Kaken Pharmaceutical Co., Ltd. and from Taito K. K. already. Examples of the process for producing the same are the methods mentioned in Japanese Patent Publication Nos. 52/3634 B and 52/44634 B.

A processed product of mycelia of *Cortinellus shiitake* such as LEM and a processed product of mycelia of *Fomos glaucotus* (*reishi*) such as MAK have been commercially available from Noda Shokkin Kogyo K. K. already. An example for the manufacturing methods thereof is that rice bran is added to bagasse (residue after squeezing of sugarcane) and mixed well, water content is adjusted and the mixture is charged in a predetermined container to prepare a solid medium followed by subjecting to sterilization by a high-pressure steam. Previously cultured various mycelia are inoculated to the medium and the mycelia are cultured in an incubating chamber of 23°C for 4 months. The medium where the mycelia abundantly grow is pulverized, autolysis is carried out and, in the case of a processed product of mycelia of *Cortinellus shiitake* and a processed product of mycelia of *Fomos glaucotus*, extraction with warm water is carried out for 15 hours. The extract is sterilized using a membrane filter and the filtrate is concentrated and dried to give powder (Japanese Patent Publication No. 7/1435 B; Japanese Patent Laid-Open No. 1/312980; Japanese Patent Publication No. 51/19013 B; and Japanese Patent Publication No. 53/18591 B).

A processed product of mycelia of *Schizophyllum commune* is a component of mycelia and is soluble in oil. Therefore, it is extracted with an appropriate organic solvent such as acetone, the extract is sterilized using a filter and the filtrate is concentrated and dried to give its powder. In the present invention, a preparation thereof for oral administration (SCP) (Yugen Kaisha Tozai Iyaku Kenkyusho) was used. Other processed products of mycelia of mushroom are also able to be prepared by the similar treatment depending upon the characteristic of the solubility thereof (whether soluble in water or in oil).

Preparation for oral administration can be made into tablets, diluted powder, capsules, syrup, etc. It is also possible of course that the preparation is prepared by a conventional means after compounding with known and necessary additives such as filler, disintegrator, binder or lubricant. If further necessary, it is also possible to add corrigent, coloring agent, perfume, stabilizer, bactericide, antiseptic agent, etc.

A therapeutic agent for cancer according to the present invention contains an effective amount of a composition of the present invention which activates the NKT cells and/or a composition of the present invention which induces the production of IL-12 and is administered orally, intravenously or intramuscularly. Oral administration is particularly preferred because a continuous self-control by a patient is possible.

A supplementary food preparation for health by oral administration according to the present invention contains an effective amount of a composition of the present invention which activates the NKT cells and/or a composition of the present invention which induces the production of IL-12 is a preparation where an anticancer effect can be expected as a result of the administration.

As mentioned hereinabove, the present invention provides a novel composition for inducing the production of IL-12 and a novel composition for activating the NKT cells and, further, it makes the relation between the IL-12 production inducing ability and the activating ability of NKT cells and also the relation between the IL-12 production inducing composition and the IL-12 production inducing ability depending upon the stage of progress of cancer clear. Accordingly, when such a fact is carried on a commercial carrier, it constitutes a discriminating means for the value of the said product. Therefore, a product where such information is carried on a commercial carrier is very highly useful. In addition, when such information is utilized in commerce, it constitutes a discriminating means for the value of the said product whereby a commercial method utilizing such information is very highly useful.

As a result, according to the present invention, a novel immunotherapy is able to be provided as a system. For example, it is possible to establish a system for carrying out immunotherapy under the tie-up and cooperation among an organization for testing the immune function, an organization for evaluating the immune function and an organization for therapy and/or formulation (medical organization). Then, when blood of a patient suffering from cancer is collected and transmitted to the organization for testing the immune function on the basis of such a system for example, measurement of at least immune function such as IL-12, NKT activity and IFNγ is carried out at the said organization and the resulting measured result is sent to the organization for evaluating the immune function and an evaluation is carried out on the basis of the test result. As a result, a therapeutic agent or a therapeutic method which is appropriate for the immune function and for the stage of progress of cancer of the said patient suffering from cancer is selected from the above-mentioned various formulations, the information is transmitted to the organization for therapy and/or formulation (a medical organization) and an immunotherapy for cancer is carried out on the basis of such a transmition. Such a novel cancer immunotherapy system is very highly useful. In addition, the cancer immunotherapy method by the above-mentioned various formulations also has a very high usefulness which has not been available yet.

### Examples

The present invention will now be more specifically illustrated by way of the Examples although the present invention is not limited thereto but various applications are possible within such an extent that they are not out of the coverage of the technical idea of the present invention.

### Example 1

### (Preparation of sample for the measurement of inducing ability of production of IL-12)

Firstly, mono nuclear cells were separated and prepared from blood of a patient suffering from cancer. Heparin-added peripheral blood obtained from the patent suffering from cancer was mixed with a phosphate buffered saline (PBS) diluted 2-fold, layered on a Ficoll-Conray liquid (specific gravity: 1.077) and centrifuged at 400G for 20 minutes to collect a mono nuclear cell layer. After washing, an RPMI-1640 medium to which a 10% fetal bovine serum (FBS) was added thereto to make the mono nuclear cell numbers 1 × 10⁶. To 200 µl of the resulting liquid where the cells were floated was added phytohemagglutinin (hereinafter, abbreviated as PHA) (manufactured by Difco) to make its concentration 20 µg/ml and incubated at 37°C for 24 hours in an atmosphere of 5% CO₂ in a 96-well microplate to prepare a sample for the measurement of cytokine in the said incubated cell solution.

### (Measurement of IL-12)

IL-12 was measured by an ELIZA using a kit manufactured by R&D Systems. Practically, 50 µl of an Assay Diluent RF1F (a diluted liquid for the measurement) and 200 µl of a standard solution or a sample prepared in Example 1 were added to each well of a 96-well microplate and made to react for 2 hours by allowing to stand at room temperature. After that, each 200 µl of anti-IL-12 antibody labeled with horse radish peroxidase (hereinafter, abbreviated as HRP) were added thereto and allowed to stand at room temperature for 2 hours. The reaction solution in each well was removed and washed for three times, each 200 µl of a coloring substrate solution were added thereto and allowed to stand at room temperature for 20 minutes and each 50 µl of a solution for stopping the enzymatic reaction were added. Absorbance of each well at 450 nm was measured by an Emax (Wako Pure Chemical Co., Ltd.) using 550 nm as a control.

### (Measurement of NKT cells)

Rate of the cells which were positive for CD3 and CD161 which are cell surface antigens was measured by a conventional manner by a Two Color test using a flow cytometry for the cells in blood using the blood of the patient suffering from cancer. With regard to monoclonal antibodies against CD3 and CD161, there were used CD3-PC5 manufactured by COULTER and CD161 manufactured by Becton-Dickinson, respectively.

### (Therapy of cancer by administration of BCG live vaccine)

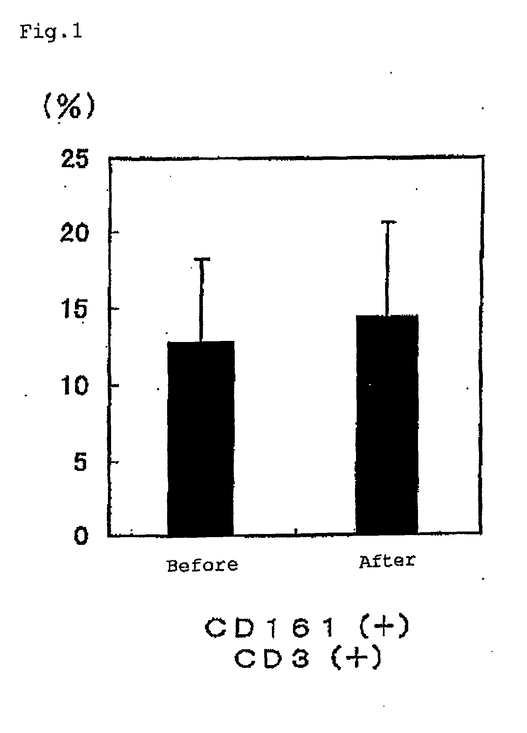

To 19 cases in total of the patients (5 cases of lung cancer, 1 case of pulmonary adenomatosis, 1 case of thymoma, 1 case of thyroid cancer, 1 case of bladder cancer, 2 cases of colon cancer, 3 cases of rectum cancer, 1 case of cecum cancer, 1 case of urinary tract cancer, 1 case of breast cancer, 1 case of uterine cervix cancer and 1 case of duplicated cancer) where IL-12 or IFNγ in incubated liquid of peripheral mono nuclear cells was in a low value in spite of continuous administration of 6 g/day of AHCC whereby a therapeutic effect was not achieved or where the therapeutic effect was not achieved in spite of the fact the IL-12 was well induced were orally administered 80 mg of BCG live vaccine (Nippon BCG Seizo K. K.) dissolved in 20% glucose with interval of one week for four times at 30 minutes to 1 hour after taking the meal. After administration for 4 times, NKT cells in blood were measured and the effect by administration of BCG live vaccine was checked. After that, the same dose as above was orally administered with interval of one month, NKT cells were measured every two months and the effect was checked. As a result, administration of the BCG live vaccine showed activation of NKT cells (Fig. 1). When the cut-off value of the rate of NKT cells in mono nuclear cells in blood was set 10%, numbers of patients suffering from cancer where the rate of NKT cells were 10% or more were 9 cases (47.3%) among 19 cases while, after administration of BCG live vaccine, it increased to 16 cases (84.2%). Further, as a result of administration of BCG live vaccine, effect of involution of the cancer was noted.

### Example 2

In place of the BCG live vaccine of the above Example 1, an SCP preparation for oral administration (Yugen Kaisha Tozai Iyaku Kenkyusho) was administered *per os* to patients suffering from cancer with interval of two weeks at the dose of 6 g/day/patient. Period for the administration was 3 months. After 3 months from the initiation of the treatment, NKT cells were measured and the effect was checked. When the cut-off value of the rate of NKT cells in mono nuclear cells in blood was set 10%, numbers of patients suffering from cancer where the rate of NKT cells were 10% or more were 8 cases among 15 cases before the administration while, after the administration, it increased to 11 cases. Further, as a result of administration of SCP, effect of involution of cancer was noted.

### Example 3

A compounded composition (IL-X) (1 kg) comprising 40 parts by weight of a processed product of mycelia of *Schizophyllum commune* (SCP; Yugen Kaisha Tozai Iyaku Kenkyusho), 40 parts by weight of a processed product of mycelia of *Cortinellus shiitake* (LEM (registered trade mark); Noda Shokkin Kogyo K. K.) and 20 parts by weight of a processed product of mycelia of *reishi* (bracket fungus of the genus *Fomos)* (MAK; Noda Shokkin Kogyo K. K.) was uniformly compounded and a granular preparation wherein three kinds were compounded was prepared by means of a fluidized granulation method by a spray drying system. The granular preparation was orally administered to patients suffering from cancer at a dose of 6 g/day/patient. Period of administration was 3 months. NKT cells and IL-12 were measured by the same manner as in Example 1 before the initiation of the treatment and after 1 month and 3 months from the initiation and the effect was checked. The result was that, among 10 cases in total of the cases where activation of NKT cells and induction of IL-12 production were confirmed, a significant involution effect of cancer was noted in 8 cases wherein 3 cases showed a complete involution of cancer.

### Example 4

A compounded composition (IL-X) (1 kg) comprising 40 parts by weight of a processed product of mycelia of *Schizophyllum commune* (SCP), 40 parts by weight of a processed product of mycelia of *Cortinellus shiitake* (LEM; Noda Shokkin Kogyo K. K.) and 20 parts by weight of a processed product of mycelia of *reishi* (bracket fungus of the genus *Fomos)* (MAK; Noda Shokkin Kogyo K. K.) was uniformly compounded, the compounded powder was made into fine granules by means of a wet granulating method and each 3 g of the said granules were charged into a capsule to prepare a capsule preparation. The capsule preparation was orally administered to patients suffering from cancer at a dose of 2 capsules/day/patient. Period of administration was 3 months. The result was that the same usefulness as in the case of Example 3 was confirmed.

### Experimental Example 1

Male cancer-carrying B10 mice (Th1) each having a body weight of 20 ± 1 g were used where one group comprised ten mice and an inducing ability for production of IL-12 (pg/ml) by various processed products of mycelia into serum was investigated. When 2 × 10⁶ cells of Lewis lung cancer were subcutaneously transplanted to the B10 mice, amount of IL-12 started to increase from the 7th day from the transplantation of cancer in the cancer-carrying mice to which no agent was administered but gradually decreased after its peak on the tenth day and, on the 18th day, it was lower than the time when cancer was transplanted. In progressive cancer or terminal cancer in human being, there are many cases where the IL-12 lowers as such *in vivo*. In the cancer-carrying living body of the progressive stage, a Th2 cytokine production pattern is noted. Therefore, in the experimental system using the said cancer-carrying B10 mice, the inducing ability for the production of IL-12 by the processed product of mycelia according to the present invention was investigated. With regard to the processed product of mycelia, each of the processed product of mycelia of *reishi* (bracket fungus of the genus *Fomos)* (MAK), a processed product of mycelia of *Cortinellus shiitake* (LEM), a processed product of mycelia of *Schizophyllum commune* (SCP), IL-X prepared in Example 3, a processed product of mycelia of *Cortinellus shiitake* (AHCC) and a processed product of mycelia of *Ganoderma boninense* (SM) was orally administered from the date when cancer was transplanted at the dose of 1 g/kg/day. On the 7th day, 10th day and 14th day after the administration, serum was collected and an induced amount of IL-12 (pg/ml) was measured. As a result, it was confirmed that, as shown in Table 1, each of the processed products of mycelia showed a characteristic and selective inducing ability with a lapse of time or, in other words, corresponding to the stage of progress of cancer to the cancer-carrying mice.

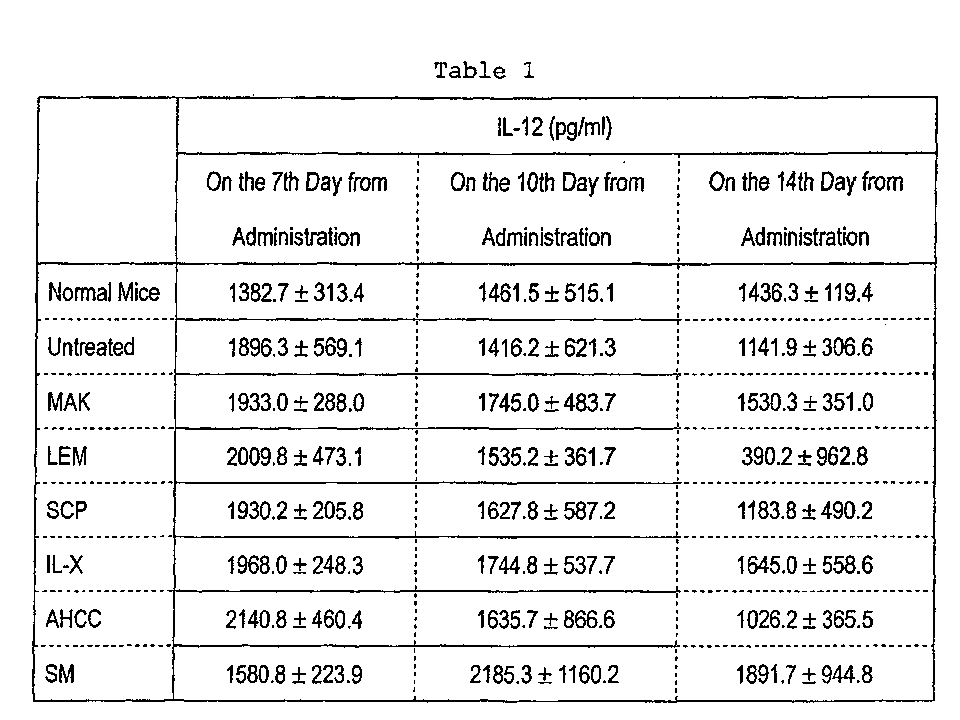

The processed product of mycelia of *glaucotus* such as a processed product of mycelia of *reishi* (bracket fungus of the genus *Fomos)* (MAK) and a processed product of mycelia of *Ganoderma boninense* (SM) achieved an inducing ability in a Th2 cytokine cascade and, particularly in terminal cancer, an inducing ability for the product of IL-12 was confirmed. The processed products of mycelia of *Cortinellus shiitake* (AHCC) (LEM) and the processed product of mycelia of *Schizophyllum commune* (SCP), an inducing ability was achieved in a Th1 cytokine cascade and, in the primary cancer, an inducing ability for IL-12 production was confirmed. A compounded composition IL-X of them was confirmed to have a function where IL-12 production was able to be induced continuously regardless of the stage of progress of cancer and to have a very high usefulness.

### Clinical Example 1

AHCC (a processed product of mycelia of *Cortinellus shiitake*) was orally administered to patients suffering from cancer 3∼6 g per day and its IL-12 production inducing ability, NKT cell activating ability and anticancer effect were tested.

It has been known that, when 7.8 pg/ml or more of IL-12 are produced, killer T cells are activated and that the more the amount of IL-12, the more significant the involution of cancer. On the other hand, with regard to the activity of NKT cells, it is judged that, when the rate of CD3(+) × CD161(+) exceeds 10%, activation takes place. Tables 2∼8 are the results of the tests for the cases.

The result was that, in the cases of pulmonary cancer by AHCC (1), gastric cancer by AHCC (2), ovarian cancer by AHCC (4) and colon cancer by AHCC (5), IL-12 in all cases was not more than 7.8 pg/ml before the treatments and, when 3∼6 g/day of AHCC were administered, IL-12 was firstly produced. As a result, tumor marker lowered and an improvement was noted clinically as well.

In the cases of duplicate of hepatic and gastric cancer by AHCC (3), productivity of IL-12 once decreased but, when productivity of IL-12 increased thereafter, tumor marker lowered. In the case of duplicated cancer by AHCC (6), productivity of IL-12 once decreased as well but, when the amount increased, tumor marker lowered. In the case of AHCC (7) (gastric cancer), IL-12 was produced but, as the NKT cells (CD3(+) × CD161(+)) increased from 13.9% to 18.3%, tumor marker lowered.

From the above, it was proved that, although AHCC has an inducing action for the production of IL-12, activating action for NKT cells was noted as well in different cases.

**Table 2**

| AHCC (1) Lung (Pulmonary) Cancer | | | | | | | | | |
|---|---|---|---|---|---|---|---|---|---|
| Date | TH1/TH2 (CD4) Ratio (≥ 7) | TNFα (≥1000 pg/ml) | IFNγ (≥ 10 IU/ml) | IL-12 (≥ 7.8 pg/ml) | CEA (Solid Phase Method) (≤ 2.5 ng/ml) | CA15-3 (≤ 30 U/ml) | BCA 225 (≤ 160 U/ml) | NSE (RIA) (≤ 10 ng/ml) | 1CTP (≤ 4.5 ng/ml) |
| 990111 | | 970 | 3 | <7.8 | 5.2 | 45 | 210 | 20 | 4.7 |
| 990312 | | | | | 1.5 | 25 | 140 | 11 | 2.8 |
| 990508 | 23 | 8040 | 38.3 | 13.9 | 1.2 | 26 | 120 | 10 | 5 |
| 990625 | | | | | 1.2 | 22 | | 7.1 | 3.5 |
| 990724 | 19.7 | 8080 | 78.5 | 89.6 | 1.3 | 24 | 130 | | 2.7 |

**Table 3**

| AHCC (2) Gastric Cancer | | | | | | | | |
|---|---|---|---|---|---|---|---|---|
| Date | TNFα (≥1000 pg/ml) | IFNγ (≥ 10 IU/ml) | IL-12 (≥ 7.8 pg/ml) | IAP (≤ 500 µg/ml) | DUPAN-2 (≤ 150 U/ml) | CA 19-9 (≤ 37 U/ml) | STN Antigen (≤ 45 U/ml) | 1CTP (≤ 4.5 ng/ml) |
| 980228 | 5820 | 112 | <7.8 | 370 | | 270 | | |
| 980502 | 2010 | 3 | <7.8 | 176 | 360 | 240 | 37 | |
| 980801 | 1220 | 28.3 | 8.1 | 67 | 140 | 66 | 37 | 10 |
| 981107 | 1360 | 15.9 | 11.2 | 54 | 100 | 58 | 41 | 7.8 |

**Table 4**

| AHCC (3) Duplicated Cancer | | | | | | |
|---|---|---|---|---|---|---|
| Date | TNFα (≥1000 pg/ml) | IFNγ (≥ 10IU/ml) | IL-12 (≥ 7.8 pg/ml) | TAP (≤ 70 U/l) | BCA 225 (≤ 160 U/ml) | 1CTP (≤ 4.5ng/ml) |
| 980227 | 3140 | 13.9 | 22.6 | 32 | 120 | 6.1 |
| 980420 | 530 | 3 | 9.7 | | | 5.6 |
| 980629 | | | | | | 4.3 |
| 980817 | | | | 24 | | 4.3 |
| 980914 | 4680 | 42.4 | 85.4 | | | 4.9 |
| 981207 | 5740 | 45.1 | 44.2 | 15 | 93 | 4.7 |

**Table 5**

| AHCC (4) Ovarian Cancer | | | | | | |
|---|---|---|---|---|---|---|
| Date | TNFα (≥1000 pg/ml) | IFNγ (≥ 10IU/ml) | IL-12 (≥ 7.8 pg/ml) | IAP (≤ 500 µg/ml) | CA 15-3 (≤ 30 U/ml) | CA 125 (≤ 35U/ml) |
| 971025 | | | | 337 | 25 | 4 |
| 971119 | 4590 | 5.2 | <7.8 | 281 | 14 | 6 |
| 971224 | | | | 316 | 11 | 7 |
| 980101 | | | | 217 | | 6 |
| 980218 | 3360 | 59.3 | 34.2 | 312 | 9.7 | 10 |
| 980325 | | | | 174 | | 8 |
| 980629 | 2990 | 34.7 | 19.9 | 197 | 8.7 | 6 |

**Table 6**

| AHCC (5) Colon Cancer | | | | | | | |
|---|---|---|---|---|---|---|---|
| Date | TH1/TH2 (CD4) Ratio (≥ 7) | TNFα (≥1000 pg/ml) | IFNγ (≥ 10 IU/ml) | IL-12 (≥ 7.8 pg/ml) | IAP (≤ 500 µg/ml) | CEA (Solid Phase Method) (≤ 2.5 ng/ml) | STN Antigen (≤ 45U/ml) |
| 981102 | | 1600 | 3.1 | <7.8 | 345 | 0.7 | 25 |
| 990109 | | 2830 | 24.1 | 12.8 | 345 | 0.5 | 22 |
| 990326 | 3.9 | 3150 | 7.7 | 22.1 | | | |

**Table 7**

| AHCC (6) Duplicated Cancer | | | | | | | | |
|---|---|---|---|---|---|---|---|---|
| Date | TNFα (≥1000 pg/ml) | IFNγ (≥ 10IU/ml) | IL-12 (≥ 7.8 pg/ml) | IAP (≤ 500 µg/ml) | SCC (≤ 1.5 ng/ml) | Sifra (CK19 Fragment) (≤ 3.5ng/ml) | BFP (≤ 75 ng/ml) | 1CTP (≤ 4.5 ng/ml) |
| 971213 | 2690 | 8.4 | 8.3 | 1050 | 1.2 | 3.6 | 160 | 7.2 |
| 980309 | 5290 | 39.2 | 17 | 317 | 1.1 | 2.2 | 49 | 6.9 |
| 980620 | 5870 | 85.7 | <7.8 | 321 | 0.7 | 1.4 | 61 | 5.5 |
| 980822 | | | | 268 | 0.9 | 2.2 | 67 | 4.8 |
| 981107 | 2350 | 53.9 | 19.5 | 362 | 0.6 | 1.4 | 63 | 4.3 |

**Table 8**

| AHCC (7) Gastric Cancer | | | | | | | | |
|---|---|---|---|---|---|---|---|---|
| Date | TH1/TH2 (CD4) Ratio (≥7) | CD 161(+) CD3(+) (≥10%) | TNFα (≥ 1000 pg/ml) | IFNγ (≥ 10 IU/ml) | IL-12 (≥ 7.8 pg/ml) | STN Antigen (≤ 45 U/ml) | 1CTP (≤ 4.5 ng/ml) | Soluble IL-2 Receptor (≤220∼530 U/ml) |
| 991106 | 8.3 | 13.9 | 1540 | 11.1 | <7.8 | 41 | 6.2 | 666 |
| 991204 | | | | | | 44 | 6 | 518 |
| 000108 | | | | | | | 5.8 | 537 |
| 000205 | 9.5 | 18.3 | 620 | 5.7 | <7.8 | 33 | 6.1 | 518 |

### Clinical Example 2

PSK (Krestin; a processed product of mycelia of *Coriolus versicolor*) was orally administered to patients suffering from cancer at a dose of 3 g per day and its IL-12 production inducing ability, NKT cell activating ability and anticancer effect were tested.

Krestin (PSK) was additionally administered to the cases where induction of production of IL-12 was not noted even when AHCC was administered. AHCC was able to induce the production of IL-12 in the cases of relatively primary cancer or the case where tumor mass was able to be removed but, in lung cancer where the progress was quick and in the cases of progressive and terminal cancers, inducing ability for the production of IL-12 significantly lowered. Accordingly, administration of PSK was necessary for the cases of progressive cancer. Tables 9∼16 show the result thereof.

In all of the six cases of PSK (1)∼(6) before the therapy, IL-12 was not produced or a reduction in the productivity was noted. However, after the administration of PSK, productivity of IL-12 was potentiated and various tumor markers were lowered whereby cancer was involuted. Although production of IL-12 was not noted in PSK (7) and PSK (8), activation (10% or more) of NKT cells was noted during the administration of PSK and various tumor markers were bettered. Accordingly, in the six cases of PSK (1)∼(6), productivity of IL-12 increased whereby it was judged that killer T cells were activated and clinical improvement was achieved. On the other hand, in PSK (7) and PSK (8), although the productivity of IL-12 was not improved by administration of PSK, the Th1/Th2 ratio improved to an extent of not less than 7.0 and tumor markers were significantly bettered as well. This is believed to be due to the fact that the NKT cells (CD3(+) × CD161(+)) were potentiated by the administration of PSK.

Accordingly, it will be concluded that PSK has an ability of inducing the production of IL-12 and an ability of activation of NKT cells.

**Table 9**

| PSK (1) Breast Cancer | | | | | | | | |
|---|---|---|---|---|---|---|---|---|
| Date | TH1/TH2 (CD4) Ratio (≥ 7) | TNFα (≥1000 pg/ml) | IFNγ (≥ 10 IU/ml) | IL-12 (≥ 7.8 pg/ml) | IAP (≤ 500 µg/ml) | BCA 215 (≤ 160 U/ml) | 1CTP (≤ 4.5 ng/ml) | Soluble IL-2 Receptor (≤ 220∼530 U/ml) |
| 990727 | 5 | 8060 | 51.8 | 18.6 | 519 | 30 | 6 | 536 |
| 990823 | | | | | 285 | | 3.8 | 408 |
| 990925 | 11.6 | 2780 | 89.6 | 79.5 | 304 | 30 | 4.5 | 310 |
| 991029 | | | | | 291 | 30 | 3.7 | 284 |

**Table 10**

| PSK (2) Breast Cancer | | | | | | | |
|---|---|---|---|---|---|---|---|
| Date | TH1/TH2 (CD4) Ratio (≥ 7) | TNFα (≥1000 pg/ml) | IFNγ (≥ 10 IU/ml) | IL-12 (≥ 7.8 pg/ml) | IAP (≤ 500 µg/ml) | CA 15-3 (≤ 30 U/ml) | BCA 225 (≤ 160 U/ml) |
| 980105 | | 3000 | 32.6 | <7.8 | 275 | 28 | 120 |
| 980411 | | 1850 | 20.5 | <7.8 | 203 | 32 | 110 |
| 980801 | | 610 | 34.4 | 11.5 | 233 | 27 | 110 |
| 990317 | 6.6 | 3030 | 50.6 | 15.1 | 207 | 27 | 89 |

**Table 11**

| PSK (3) Gastric Cancer | | | | | | | | |
|---|---|---|---|---|---|---|---|---|
| Date | TH1/TH2 (CD4) Ratio (≥ 7) | TNFα (≥1000 pg/ml) | IFNγ (≥ 10 IU/ml) | IL-12 (≥ 7.8 pg/ml) | IAP (≤ 500 µg/ml) | CA72-4 (≤ 4.0 U/ml) | STN Antigen (≤ 45U/ml) | Soluble IL-2 Receptor (≤ 220∼530 U/ml) |
| 990206 | 1.5 | 1420 | 4 | 14.8 | 518 | 6.2 | 31 | 1520 |
| 990222 | | | | | 573 | 4.5 | 35 | 1800 |
| 990326 | | | | | 371 | 5.1 | 36 | 1180 |
| 990508 | 5.6 | 1550 | 12.4 | 20.9 | 543 | 3 | 40 | 893 |
| 990619 | | | | | 416 | 3.1 | 37 | 827 |
| 990726 | 3.5 | 2670 | 13.8 | 48.9 | | 4.9 | 39 | 884 |

**Table 12**

| PSK (4) Breast Cancer | | | | | | |
|---|---|---|---|---|---|---|
| Date | TNFα (≥1000 pg/ml) | IFNγ (≥ 10IU/ml) | IL-12 (≥ 7.8 pg/ml) | IAP (≤ 500 µg/ml) | NCC-ST-439 (≤7.0 U/ml) | CA 15-3 (≤ 30U/ml) |
| 980126 | 6250 | 12.8 | <7.8 | 292 | 22 | 48 |
| 980302 | | | | 275 | 7.9 | 23 |
| 980323 | 8170 | 44.7 | 21.8 | 298 | 5.3 | 27 |
| 980427 | | | | 238 | 3.1 | 21 |
| 980525 | | | | 209 | 2.2 | 17 |
| 980620 | 2620 | 9.5 | <7.8 | 231 | 1.6 | 16 |

**Table 13**

| PSK (5) Testicular Tumor | | | | | | | | | |
|---|---|---|---|---|---|---|---|---|---|
| Date | TH1/TH2 (CD4) Ratio (≥ 7) | TNFα (≥1000 pg/ml) | IFNγ (≥ 10 IU/ml) | IL-12 (≥ 7.8 pg/ml) | Qantitative Detn of α-Fet protein (≤ 20 ng/ml) | HCG (EIA) (≤ 0.7 mlU/ml) | HCGβ in blood (≤ 0.1 ng/ml) | 1CTP (≤ 4.5 ng/ml) | Soluble IL-2 Receptor (≤ 220∼530 U/ml) |
| 990313 | 6.7 | 710 | 7 | <7.8 | 2.9 | <0.4 | <0.1 | 7.7 | 672 |
| 990424 | | | | | | <0.4 | <0.1 | 3.7 | 489 |
| 990522 | 8.6 | 1220 | 8.3 | <7.8 | 1.5 | <0.4 | <0.1 | 5 | 456 |
| 990625 | | | | | | | | 4.5 | 424 |
| 990724 | | | | | 1.5 | <0.4 | <0.1 | 3.6 | 335 |
| 990820 | 9.8 | 2040 | 15.7 | 28.1 | <1.0 | <0.4 | <0.1 | 2.8 | 324 |

**Table 14**

| PSK (6) Brain Tumor | | | | | | | |
|---|---|---|---|---|---|---|---|
| Date | TH1/TH2 (CD4) Ratio (≥7) | TNFα (≥1000 pg/ml) | IFNγ (≥ 10 IU/ml) | IL-12 (≥ 7.8 pg/ml) | IAP (≤ 500 µg/ml) | 1CTP (≤ 4.5ng/ml) | Soluble IL-2 Receptor (≤ 220∼530 U/ml) |
| 990305 | 7.4 | 590 | 2.9 | <7.8 | 269 | 5.7 | 631 |
| 990416 | | | | | | 5.7 | 780 |
| 990604 | 9.6 | 1520 | 6.2 | <7.8 | | 5.6 | 435 |
| 990727 | | | | | | 5.2 | 382 |
| 990924 | 8.9 | 2800 | 38.5 | 14.6 | 225 | 3.4 | 399 |

**Table 15**

| PSK (7) Malignant Lymphoma | | | | | | | |
|---|---|---|---|---|---|---|---|
| Date | TH1/TH2 (CD4) Ratio (≥ 7) | CD 161(+) CD3(+) (≥ 10%) | TNFα (≥ 1000 pg/ml) | IFNγ (≥ 10 IU/ml) | IL-12 (≥ 7.8 pg/ml) | 1CTP (≤ 4.5ng/ml) | Soluble IL-2 Receptor (≤ 220∼530 U/ml) |
| 990717 | 4.2 | 7.2 | 2040 | 5.2 | <7.8 | 7 | 720 |
| 990814 | | | | | | 7.2 | 588 |
| 990911 | | | | | | 6.4 | 434 |
| 991015 | 26.6 | 14.2 | 3180 | 47.6 | <7.8 | 6.9 | 424 |
| 991127 | | | | | | 5.5 | 429 |
| 991218 | | | | | | 5.5 | 366 |
| 000122 | 23.5 | 18.5 | 2410 | 37.7 | <7.8 | 5.1 | 346 |
| 000219 | | | | | | 4.4 | 346 |

**Table 16**

| PSK (8) Gastric Cancer (scirrhous) | | | | | | | | |
|---|---|---|---|---|---|---|---|---|
| Date | TH1/TH2 (CD4) Ratio (≥ 7) | CD 161(+) CD3(+) (≥ 10%) | TNFα (≥ 1000 pg/ml) | IFNγ (≥ 10 IU/ml) | IL-12 (≥ 7.8 pg/ml) | CEA (solid phase method) (≤ 2.5 ng/ml) | 1CTP (≤ 4.5 ng/ml) | Soluble IL-2 Receptor (≤ 220-530 U/ml) |
| 991224 | 4.2 | 12.5 | 1320 | 7.7 | <7.8 | 6.9 | 4.4 | 1780 |
| 000121 | | | | | | 5.8 | 3.4 | 730 |
| 000218 | | | | | | 3.2 | 3.9 | 814 |
| 000317 | 15.8 | 17.2 | 1070 | 10.9 | <7.8 | 2.4 | 5.3 | 300 |

### Clinical Example 3

SPG (a polysaccharide derived from cultured filtrate of mycelia of *Schizophyllum commune*) (2 mg/ampoule) was intramuscularly administered to patients suffering from cancer and its IL-12 production inducing ability, NKT cell activating ability and anticancer effect were tested. The results are shown in Tables 17∼28.

SPG was intramuscularly administered to the cases where no clinical improvement by administration of AHCC and PSK (Krestin) was noted and neither induction of IL-12 production nor activation of NKT cells was potentiated. With regard to the administration of SPG, administering times were varied within twice a week to once a month depending upon the symptoms.

In the 8 cases of SPG (1)∼(8), productivity of IL-12 was not more than 7.8 pg/ml or lowered. By the administration of SPG, productivity of IL-12 was significantly improved, various tumor markers were significantly lowered and clinical improvement was noted as well. In the 4 cases of SPG (9)∼(12), productivity of IL-12 was improved but potentiation of activation of NKT cells was noted as well (10% or more) whereby various tumor markers lowered and clinical improvement was noted as well.

Accordingly, it was found that SPG had an inducing ability for the production of IL-12 and an activating ability for NKT cells.

**Table 17**

| SPG (1) Cecum Cancer; SPG Injection; Started on December 14, 1998 | | | | | | | |
|---|---|---|---|---|---|---|---|
| Date | TH1/TH2 (CD4) Ratio (≥ 7) | TNFα (≥1000 pg/ml) | IFNγ (≥ 10 IU/ml) | IL-12 (≥ 7.8 pg/ml) | CEA (Solid Phase Method) (≤ 2.5 ng/ml) | CA 72-4 (≤ 4 U/ml) | STN Antigen (≤ 45 U/ml) |
| 981214 | | 2460 | 7.2 | <7.8 | 1.2 | 6.3 | 54 |
| 990123 | | | | | | 9.2 | 48 |
| 990215 | | | | | | 11 | 47 |
| 990703 | 26.3 | 1110 | 19.2 | 15.5 | 0.8 | 3 | 29 |

**Table 18**

| SPG (2) Malignant Lymphoma; SPG Injection; Started on March 6, 1999 | | | | | | |
|---|---|---|---|---|---|---|
| Date | TH1/TH2 (CD4) Ratio (≥ 7) | TNFα (≥1000 pg/ml) | IFNγ (≥ 10 IU/ml) | IL-12 (≥ 7.8 pg/ml) | CA 125 (≤ 35 U/ml) | STN Antigen (≤ 45U /ml) |
| 990306 | 6.1 | 2830 | 43.8 | 19 | 96 | |
| 990409 | | | | | 77 | 27 |
| 990611 | 4.1 | 6180 | 50.2 | 43.3 | 64 | 19 |
| 990710 | | | | | 59 | |
| 990903 | 7.9 | 3640 | 44.9 | 30.3 | 43 | |

**Table 19**

| SPG (3) Colon Cancer; SPG Injection; Started on November 9, 1998 | | | | | | | |
|---|---|---|---|---|---|---|---|
| Date | TH1/TH2 (CD4) Ratio (≥7) | TNFα (≥1000 pg/ml) | IFNγ (≥ 10 IU/ml) | IL-12 (≥ 7.8 pg/ml) | CA 19-9 (≤ 37 U/ml) | STN Antigen (≤45 U/ml) | 1CTP (≤ 4.5 ng/ml) |
| 981109 | | 3160 | 37.1 | 28 | 68 | 34 | 3.4 |
| 981226 | | | | | 80 | | |
| 990227 | 7.7 | 1660 | 121 | 116 | 54 | 32 | 3.2 |
| 990326 | | | | | 49 | | 2.9 |

**Table 20**

| SPG (4) Breast Cancer; SPG Injection; Started on May 29 1999 | | | | | | | |
|---|---|---|---|---|---|---|---|
| Date | TH1/TH2 (CD4) Ratio (≥ 7) | TNFα (≥1000 pg/ml) | IFNγ (≥ 10 IU/ml) | IL-12 (≥ 7.8 pg/ml) | BCA 225 (≤ 160 U/ml) | 1CTP (≤ 4.5 ng/ml) | Soluble IL-2 Receptor (≤ 220∼530 U/ml) |
| 990529 | 6.3 | 1114 | 14.8 | <7.8 | | 4.5 | 575 |
| 990626 | | | | | 140 | 5.1 | 701 |
| 990724 | | | | | 150 | 5.1 | 563 |
| 990820 | 8.1 | 3520 | 74.4 | 61.6 | 110 | 3.7 | 442 |

**Table 21**

| SPG (5) Hepatic Cancer; SPG Injection; Started on June 6, 1998 | | | | | |
|---|---|---|---|---|---|
| Date | TNFα (≥1000 pg/ml) | IFNγ (≥ 10IU/ml) | IL-12 (≥7.8 pg/ml) | IAP (≤ 500 µg/ml) | PIVKA-2 (≤ 40 mAU/ml) |
| 980606 | 4880 | 37.8 | 8.3 | 242 | 163 |
| 980711 | | | | 377 | 340 |
| 980808 | | | | 203 | 40 |
| 980905 | 1130 | 102 | 35.8 | 210 | 31 |

**Table 22**

| SPG (6) Cholecyst Cancer; SPG Injection; Started on August 23, 1999 | | | | | | |
|---|---|---|---|---|---|---|
| Date | TH1/TH2 (CD4) Ratio (≥ 7) | TNFα(≥1000 pg/ml) | IFNγ (≥ 10IU/ml) | IL-12 (≥ 7.8 pg/ml) | CA72-4( ≤ 4 U/ml) | 1CTP (≤ 4.5ng/ml) |
| 990823 | 4.9 | 970 | 4.5 | <7.8 | 7.7 | 5 |
| 991002 | | | | | 11 | 6.2 |
| 991106 | 3.7 | 1030 | 21 | 18 | 24 | 4.9 |
| 991122 | | | | | 3 | 5 |
| 000124 | 2.8 | 1240 | 33.1 | 27.1 | | 4.4 |

**Table 23**

| SPG (7) Breast Cancer; SPG Injection; Started on November 2, 1998 | | | | | | | | |
|---|---|---|---|---|---|---|---|---|
| Date | TH1/TH2 (CD4) Ratio (≥7) | TNFα (≥1000 pg/ml) | IFNγ (≥ 10 IU/ml) | IL-12 (≥7.8 pg/ml) | TPA (≤ 70 U/l) | NCC-ST-439 (≤ 7.0U/ml) | CA15-3 (≤ 30 U/ml) | CA72-4 (≤ 4.0 U/ml) |
| 980928 | | 6150 | 28.4 | 25.5 | 150 | 5.8 | 14 | 6.3 |
| 981102 | | | | | 41 | | | 4.1 |
| 981207 | | 7800 | 81.9 | 64.3 | 22 | | | 3.5 |
| 990116 | | | | | 10 | | | 3 |
| 990215 | 8.2 | 2750 | 63.6 | 61.8 | 26 | 2.9 | 11 | |
| 990402 | | | | | 17 | 1.4 | | 3 |
| 990515 | 8.1 | 3590 | 49.1 | 68.6 | | | | |
| 990618 | | | | | 23 | | | |
| 009807 | 10.2 | 1120 | 36.4 | 55.6 | 14 | 2.2 | 9.2 | |

**Table 24**

| SPG (8) Breast Cancer; SPG Injection; Started on October 23, 1999 | | | | | | | | |
|---|---|---|---|---|---|---|---|---|
| Date | TH1/TH2 (CD4) Ratio (≥ 7) | CD 161(+) CD3(+) (≥ 10%) | TNFα (≥ 1000 pg/ml) | IFNγ (≥ 10 IU/ml) | IL-12 (≥ 7.8 pg/ml) | CEA (solid phase method) (≤ 2.5ng/ml) | CA 15-3 (≤ 30 U/ml) | BCA 225 (≤ 160 U/ml) |
| 990828 | 13.6 | | 85 | 3.8 | 7.8 | 3.1 | 120 | 1600 |
| 991008 | 14.9 | | 530 | 4.5 | 7.8 | 17 | 160 | 2100 |
| 991023 | | | | | | 12 | 110 | 1700 |
| 000122 | 13.8 | 19.2 | 7810 | 256 | 38.3 | 1.2 | 26 | 180 |

**Table 25**

| SPG (9) Pancreatic Cancer; SPG Injection; Started on November 12 1999 | | | | | | | |
|---|---|---|---|---|---|---|---|
| Date | TH1/TH2 (CD4) Ratio (≥ 7) | CD 161(+) CD3(+) (≥ 10%) | TNFα (≥ 1000 pg/ml) | IFNγ (≥ 10 IU/ml) | IL-12 (≥ 7.8 pg/ml) | CA 19-9 (≤ 37 U/ml) | STN Antigen (≤ 45U/ml) |
| 991015 | 8.3 | 11.2 | 690 | 11.7 | <7.8 | 910 | 41 |
| 991112 | | | | | | 770 | |
| 991210 | 16 | 7.4 | 800 | 3.1 | <7.8 | 780 | |
| 000107 | | | | | | 130 | |
| 000324 | 9.7 | 15.4 | 2070 | 16.8 | <7.8 | 87 | 33 |

**Table 26**

| SPG (10) Lung (Pulmonary) Cancer; SPG Injection; Started on January 8, 2000 | | | | | | | | | |
|---|---|---|---|---|---|---|---|---|---|
| Date | TH1/ TH2 (CD4) Ratio (≥ 7) | CD 161(+) CD3(+) (≥ 10%) | TNFα (≥ 1000 pg/ ml) | IFNγ(≥ 10 IU/ml) | IL-12 (≥ 7.8 pg/ ml) | TPA (≤ 70 U/l) | SCC (≤ 1.5 ng/ ml) | Sifra (CK 19 Fragment) (≤ 3.5 ng/ ml) | Soluble IL-2 Receptor (≤ 220-530 U/ml) |
| 000108 | 4.1 | 16.3 | 290 | 0.8 | <7.8 | 72 | 4.8 | 4.1 | 784 |
| 000205 | 6.2 | | 710 | 11.8 | <7.8 | 45 | 1.2 | 3.5 | 1120 |
| 000311 | 24.3 | 23.8 | 92 | 0.9 | <7.8 | 46 | 0.6 | 4.8 | 792 |

**Table 27**

| SPG (11) Lung Cancer; SPG Injection; Started on December 25, 1999 | | | | | | | | | |
|---|---|---|---|---|---|---|---|---|---|
| Date | TH1/ TH2 Ratio (≥ 7) | CD 161(+) (CD4) (≥ 10%) | TNFα (≥ CD3(+) pg/ ml) | IFNγ (≥ 10 1000 IU/ml) | IL-12 (≥ 7.8 U/l) pg/ ml) | TPA (≤ 70 Phase | CEA (Solid (≤ 30 Method) (≤ 2.5 ng/ml) | CA 15-3 U/ml) | Sifra (CK 19 Fragment) (≤ 3.5 ng/ ml) |
| 991225 | 3.7 | 11.9 | 430 | 1.5 | <7.8 | 550 | 44 | 440 | 19 |
| 000122 | | | | | | 420 | 52 | 580 | 18 |
| 000218 | | | | | | 340 | 55 | 410 | 13 |
| 000318 | 2.3 | 23.2 | 360 | 4.6 | <7.8 | 89 | 36 | 160 | 4.5 |

**Table 28**

| SPG (12) Rectum Cancer; SPG Injection; Started on November 19, 1999 | | | | | | |
|---|---|---|---|---|---|---|
| Date | TH1/TH2 (CD4) Ratio (≥ 7) | CD 161(+) CD3(+) (≥ 10%) | TNFα (≥ 1000 pg/ml) | IFNγ (≥ 10 IU/ml) | IL-12 (≥ 7.8 pg/ml) | CA 19-9 (≤ 37 U/ml) |
| 991119 | 6.9 | 13.7 | 37 | 0.8 | <7.8 | 49 |
| 991211 | | | | | | 44 |
| 000114 | 2.2 | 19.5 | 138 | 1.8 | <7.8 | 16 |

### Clinical Example 4

SCP Tablets (a processed product of mycelia of *Schizophyllum commune)* was orally administered to patients suffering from cancer at a dose of 18∼36 mg per day and its IL-12 production inducing ability, NKT cell activating ability and anticancer effect were tested. The result is shown in Tables 29∼31. The SCP tablets which are the substance derived from mycelia of *Schizophyllum commune* were orally administered at a dose of from 9 to 18 tablets a day depending upon the degree of progress of cancer.

The case of SCP (1) was rectum cancer and productivity of IL-12 was 7.8 pg/ml or less in primary stage but, after one half year from the administration, it increased, various tumor markers lowered and metastasis to liver disappeared as well. The case of SCP (2) was lung cancer and, in its primary stage, IL-12 productivity was 7.8 pg/ml or less and NKT cells were somewhat promoted to an extent of 13.7% but, on the third month from the administration, potentiation of IL-production and NKT cell activation was noted, lung cancer was involuted to one half and the tumor markers were significantly improved as well. SCP (3) was prostatic cancer and no production of IL-12 was noted. However, potentiation of activation of NKT cells was noted, tumor markers were improved and metastasis to bone disappeared as well.

Accordingly, SCP can be said to have an inducing ability of IL-12 production and an activating ability for NKT cells.

**Table 29**

| SCP (1) Rectum Cancer; SCP Started on June 14, 1999 | | | | | | |
|---|---|---|---|---|---|---|
| Date | TH1/TH2 (CD4) Ratio (≥ 7) | TNFα (≥1000 pg/ml) | IFNγ (≥ 10 IU/ml) | IL-12 (≥7.8 pg/ml) | Sialyl LEX-1 (≤38 U/ml) | STN Antigen (≤ 45U/ml) |
| 990614 | | | | | 42 | 90 |
| 990809 | 5.0 | 280 | 6.5 | <7.8 | 43 | 110 |
| 991115 | | | | | 44 | 110 |
| 000124 | 8.2 | 1850 | 20.2 | 11.1 | 38 | 70 |

**Table 30**

| SCP (2) Lung Cancer; SCP; Started on January 21, 2000 | | | | | | | | |
|---|---|---|---|---|---|---|---|---|
| Date | TH1/TH2 (CD4) Ratio (≥ 7) | CD 161(+) CD3(+) (≥ 10%) | TNFα (≥ 1000 pg/ml) | IFNγ (≥ 10 IU/ml) | IL-12 (≥ 7.8 pg /ml) | CEA (Solid Phase Method) (≤ 2.5 ng/ml) | 1CTP (≤ 4.5 ng/ml) | Soluble IL-2 Receptor (≤ 220∼530 U/ml) |
| 991203 | 4.9 | 13.7 | 950 | 11.2 | <7.8 | 19 | 4.2 | 650 |
| 991224 | | | | | | 19 | 5.1 | 788 |
| 000121 | | | | | | 11 | 4.7 | 773 |
| 000218 | | | | | | 12 | 5.2 | 522 |
| 000318 | 3.9 | 16.8 | 1070 | 35 | 14.2 | 9.3 | 2.8 | 405 |

**Table 31**

| SCP (3) Prostatic Cancer; SCP; Started on February 18, 2000 | | | | | | | | |
|---|---|---|---|---|---|---|---|---|
| Date | TH1/TH2 (CD4) Ratio (≥ 7) | CD 161(+) CD3(+) (≥ 10%) | TNFα (≥ 1000 pg/ml) | IFNγ (≥ 10 IU/ml) | IL-12 (≥ 7.8 pg /ml) | PA (RIA) (≤ 4ng/ml) | γ-Semino-protein (≤ 4 ng/ml) | PAP (EIA) (≤ 3 ng/ml) |
| 991225 | 11.3 | 15.6 | 1510 | 28.9 | <7.8 | 71 | 89 | 1340 |
| 000121 | | | | | | 79 | 73 | 2410 |
| 000218 | | | | | | 18 | 23 | 190 |
| 000317 | 7.5 | 19.8 | 126 | 3.2 | <7.8 | 26 | 38 | 397 |

### Clinical Example 5

As same as in the above Example 1, IL-12 production inducing ability, NKT cell activating ability and anticancer effect by oral administration of BCG live vaccine were tested. The result is shown in Tables 32∼36.

Oral administration therapy of BCG live vaccine tends to inhibit the IL-12 productivity. Accordingly, the object for the therapy should be the cases where IL-12 productivity is not promoted. Thus, the cases where potentiation of IL-12 productivity or NKT cell activation is not available by administration of the substances derived from mycelia such as AHCC, PSK, SPG and SCP were to be the objects.

In the cases BCG (1)∼(5), a BCG live vaccine therapy was applied to the cases where potentiation of IL-12 productivity was not achieved or lowered. By the administration of BCG live vaccine, potentiation of NKT cell activation was available and tumor markers lowered as well. In addition, clinical improvement was noted in all cases.

Accordingly, BCG was found to have an NKT cell activating ability.

**Table 32**

| BCG (1) Gastric Cancer; BCG Started on December 11, 1999 | | | | | | | | | | |
|---|---|---|---|---|---|---|---|---|---|---|
| Date | TH1/ TH2 (CD4) Ratio (≥ 7) | CD 161(+) CD3(+) (≥ 10%) | TNF α (≥ 1000 pg/ ml) | IFNγ (≥ 10 IU /ml) | IL-12 (≥ 7.8 pg /ml) | CA 19-9 (≤ 37 U /ml) | CA 72-4 (≤ 4U /ml) | STN Anti-gen (≤ 45 U/ml) | 1CTP (≤ 4.5 ng/ml ) | Soluable IL-2 Receptor (≤ 220-530 U/ml) |
| 991211 | 15.2 | 14 | 2410 | 7.9 | <7.8 | 12 | 3 | 30 | 7.3 | 592 |
| 000108 | 5.7 | 11.5 | 410 | 2.9 | <7.8 | 6 | 3.4 | 31 | 5.5 | 410 |
| 000311 | 7.4 | 17.5 | 610 | 4.7 | <7.8 | 11 | 11 | 3 | 5.5 | |

**Table 33**

| BCG (2) Gastric Cancer; Started on December 11, 1999 | | | | | | | | | |
|---|---|---|---|---|---|---|---|---|---|
| Date | TH1 /TH2 (CD4) Ratio (≥ 7) | CD 161(+) CD3(+) (≥ 10%) | TNFα (≥ 1000 pg /ml) | IFNγ (≥10 IU /ml) | IL-12 (≥ 7.8 pg /ml) | CA 72-4 (≤ 4U /ml) | STN Antigen (≤ 45 U /ml) | 1CTP (≤ 4.5 ng /ml) | Sol. IL-2 Receptor (≤ 220∼ 530 U/ml) |
| 991106 | 9 | 9.8 | 1620 | 4.2 | <7.8 | 6.2 | 28 | 5.3 | 478 |
| 991211 | | | | | | 5.4 | 25 | 4.2 | 456 |
| 000108 | 7.1 | 10.3 | 220 | 2.5 | <7.8 | 3.2 | 19 | 5.6 | 373 |
| 000212 | | | | | | 3.8 | 19 | 4.7 | 456 |
| 000311 | 9.6 | 12.7 | 171 | 1.4 | <7.8 | | | 2.9 | |

**Table 34**

| BCG (3) Lung Cancer; BCG started on December 10, 1999 | | | | | | | | |
|---|---|---|---|---|---|---|---|---|
| Date | TH1/TH2 (CD4) Ratio (≥7) | CD 161(+) CD3(+) (≥ 10%) | TNFα (≥ 1000 pg/ml) | IFNγ (≥ 10 IU/ml) | IL-12 (≥ 7.8 pg/ml) | IAP (≤ 500 µg /ml) | Siaryl LEX-1 (≤ 38 U /ml) | Sol. IL-2 Receptor (≤ 220∼ 350U/ml) |
| 991210 | 11.4 | 9.3 | 550 | 6.1 | <7.8 | 321 | 29 | 468 |
| 000107 | 8.9 | 10.6 | 250 | 4.2 | <7.8 | | | |
| 000204 | | | | | | | | 416 |
| 000303 | 8.5 | 12.1 | 400 | 4 | <7.8 | 308 | 27 | 346 |

**Table 35**

| BCG (4) Thyroid Cancer; BCG started on November 22, 1999 | | | | | | | |
|---|---|---|---|---|---|---|---|
| Date | TH1/TH2 (CD4) Ratio (≥7) | CD 161(+) CD3(+) (≥ 10%) | TNFα (≥ 1000 pg/ml) | IFNγ (≥ 10 IU/ml) | IL-12 (≥ 7.8 pg/ml) | 1CTP (≤ 4.5 ng/ml) | Cycloglobulin (≤ 30 ng/ml) |
| 991122 | 5.3 | 8.5 | 2520 | 27.6 | 15 | 7.4 | 210 |
| 991217 | 6.3 | 11.9 | 1490 | 9.8 | 12 | 6.6 | 160 |

**Table 36**

| BCG (5) Thymoma; BCG started on November 22, 1999 | | | | | | | | |
|---|---|---|---|---|---|---|---|---|
| Date | TH1/TH2 (CD4) Ratio (≥ 7) | CD 161(+) CD3(+) (≥ 10%) | TNFα (≥ 1000 pg/ml) | IFNγ (≥ 10 IU/ml) | IL-12 (≥ 7.8 pg/ml) | IAP (≤ 500 µg /ml) | NSE (RIA) (≤ 10 ng/ml) | Sol. IL-2 Receptor (≤ 220∼ 530 U/ml) |
| 991122 | | 17.7 | | | | 401 | | 514 |
| 991217 | 7.4 | 15.8 | 930 | 1.4 | <7.8 | 376 | 6.6 | 438 |
| 000212 | 5.8 | 14.1 | 680 | 3.5 | <7.8 | 318 | 6 | 452 |

### Example 5

An organization for testing the immune function, a medical organization and an organization for instructing the test of immunological competence and the policy for therapy carry out a business tie-up where share of role of business for each organization is made clear.

At the medical organization, blood is collected from patients by a known blood collecting method corresponding to the aimed test measurement items in the said blood components. The medical organization also prepares serum and blood cell components from the blood if necessary depending upon the aimed test measurement items. After the medical organization identifies blood, serum and/or blood cell components and the patient wherefrom the above are collected, the organization immediately sends them to the tied-up organization for testing the immune function.

The organization for testing the immune function carries out the test for specific immune-related function (such as IL-12 productivity, NKT cell activity, IFNγ value and TNFα value) previously determined by the instruction of the organization for instructing and the policy for therapy (hereinafter, referred to as "the organization for instruction") for the blood, serum and/or blood cell components sent from the medical organization. If necessary, serum and blood components are prepared from the blood. In addition to the said test for the immune function-related function, the organization for testing the immune function carries out a test for cancer marker, cancer-related factors (such as angiogenesis promotion factor and angiogenesis inhibiting factor), etc. and advises the test results to the organization for instruction.

On the basis of the received test results, the organization for instruction carries out the tests at least for IL-12 productivity, NKT cell activity, IFNγ value and TNFα value and also carries out the tests for angiogenesis promoting factor (such as vascular endothelial growth factor : VEGF), angiogenesis inhibiting factor (such as endostatin) and, if necessary, various cancer markers. On the basis of the test results, the organization for instruction further specifies a treatment way which adapts the test results from the examples of formulations for auxiliary anticancer agents such as IL-12 inducers, NKT cell activators, other BRM (biological response modifier) preparations and shark cartilage preparations. The test result and formulation example are immediately transmitted to the tied-up medical organization.

The medical organization carries out the therapy of cancer for the patient referring to the instructed formulation example.

The organization for instruction further tests the various test results carried out for the blood components of the patient by the same method as above during the course of the therapy, judges the efficacy and inefficacy of the therapy and proposes the more appropriate formulation example adapting the test result to the medical organization. The organization for instruction receives the instruction charge by proposing the test result and the formulation example to the medical organization.

### Possibility of Industrial Usage

The present invention clarifies the causes in the patient who is still non-responsive in the cancer therapy by induction of IL-12 production and provides a useful means for further cancer therapy on the basis of the information of the cancer immune cascade. The present invention further provides a processed product of mycelia of mushroom effective to induction of IL-12 production and clarifies the effective administration period according to the stage of progress of cancer in induction of IL-12 production by the processed product of mycelia of mushroom. Consequently, the present invention is able to greatly contribute to cancer therapy.

## Claims

1. A therapeutic agent for cancer where an inducing ability for the production of interleukin 12 (IL-12) and an activating ability for NKT cells are used as indexes.

2. The therapeutic agent for cancer according to claim 1, wherein the cell ratio of Th1/Th2 being about 7.0 or more is used as an index for the effect.

3. The therapeutic agent for cancer according to claim 1, wherein the agent contains a substance having an activating ability for NKT cells which is administered to a patient where the amount of IL-12 produced *in vivo* is low even by administration of an inducing agent for the production of IL-12 or to a patient where the therapeutic effect for cancer is not achieved even when the IL-12 value is high.

4. The therapeutic agent for cancer according to any of claims 1 to 3, wherein it is a supplementary food preparation for health by oral administration.

5. A composition which activates the NKT cells where at least one selected from live cells, attenuated live cells and a thing derived from mycelia of mushroom is an active ingredient.

6. The composition according to claim 5, wherein the substance having a function of activating the NKT cells is BCG live vaccine or a thing derived therefrom.

7. The composition according to claim 5, wherein the thing derived from mycelia of mushroom having a function of activating the NKT cells is selected from a processed product of mycelia of *Schizophyllum commune,* a polysaccharide derived from cultured filtrate of mycelia of *Schizophyllum commune,* a processed product of mycelia of *Coriolus versicolor* and a processed product of mycelia of *Cortinellus shiitake*.

8. The composition according to claim 7, wherein the processed product of mycelia of *Schizophyllum commune* is SCP, the polysaccharide derived from cultured filtrate of mycelia of *Schizophyllum commune* is SPG, the processed product of mycelia of *Coriolus versicolor* is PSK (Krestin®) and the processed product of mycelia of *Cortinellus shiitake* is AHCC and LEM.

9. The composition according to claim 5, wherein the substance having a function of activating the NKT cells is a saccharide having α-1,3 and/or α-1,4 glucoside bond structure(s).

10. The composition according to claim 9, wherein the saccharide is a polysaccharide and/or an oligosaccharide.

11. A composition which is **characterized in** compounding at least a substance having an inducing ability for the production of IL-12 and a substance having a function of activating the NKT cells.

12. The composition according to claim 11, wherein at least one component selected from a processed product of mycelia of *Schizophyllum commune,* a polysaccharide derived from cultured filtrate of mycelia of *Schizophyllum commune,* a processed product of mycelia of *Cortinellus shiitake*, a processed product of mycelia of *Fomos glaucotus* and a processed product of mycelia of *Coriolus versicolor* as the substance having an inducing ability of production of IL-12 and at least one component selected from a product derived from BCG live vaccine, a processed product of mycelia of *Coriolus versicolor,* a processed product of mycelia of *Schizophyllum commune,* a polysaccharide derived from cultured filtrate of mycelia of *Schizophyllum commune* and a polysaccharide having α-1,3 and/or α-1,4 glucoside bond structure(s) as the substance having an activating ability to NKT cells are compounded.

13. The composition according to claim 12, wherein the processed product of mycelia of *Schizophyllum commune* is SCP, the polysaccharide derived from cultured filtrate of mycelia of *Schizophyllum commune* is SPG, the processed product of mycelia of *Cortinellus shiitake* is AHCC and LEM, the processed product of mycelia of *Fomos glaucotus* is SM and MAK and the processed product of mycelia of *Coriolus versicolor* is PSK (Krestin®).

14. The composition according to claim 12, wherein three kinds of substances - the processed product of mycelia of *Schizophyllum commune* or the polysaccharide derived from cultured filtrate of mycelia of *Schizophyllum commune,* the processed product of mycelia of *Cortinellus shiitake* and the processed product of mycelia of *Fomos glaucotus -* are compounded.

15. The composition according to claim 14, wherein 30∼50 parts by weight of the processed product of mycelia of *Schizophyllum commune* or the polysaccharide derived from cultured filtrate of mycelia of *Schizophyllum commune,* 30∼50 parts by weight of the processed product of mycelia of *Cortinellus shiitake* and 10∼30 parts by weight of the processed product of mycelia of *Fomos glaucotus* are compounded.

16. The composition according to claim 14 or 15, wherein the processed product of mycelia of *Schizophyllum commune* is SCP, the polysaccharide derived from cultured filtrate of mycelia of *Schizophyllum commune* is SPG, the processed product of mycelia of *Cortinellus shiitake* is AHCC or LEM, the processed product of mycelia of *Fomos glaucotus* is SM or MAK and the processed product of *Coriolus versicolor* is PSK (Krestin®).

17. A composition for inducing the production of IL-12 in which at least one which is selected from a processed product of mycelia of *reishi (*bracket fungus of the genus *Fomos)*, a processed product of mycelia of *Ganoderma boninense* and a processed product of mycelia of *Coriolus versicolor* is contained as an active ingredient.

18. The composition for inducing the production of IL-12 according to claim 17, wherein the processed product of mycelia of *reishi (*bracket fungus of the genus *Fomos)* is MAK, the processed product of mycelia of *Ganoderma boninense* is SM and the processed product of *Coriolus versicolor* is PSK (Krestin ®).

19. The composition for inducing the production of IL-12 according to claim 17 or 18, wherein it is administered to a patient suffering from progressive or terminal cancer.

20. A composition for inducing the production of IL-12 to be administered to a patient suffering from primary cancer in which at least one which is selected from a processed product of mycelia of *Schizophyllum commune,* a polysaccharide derived from cultured filtrate of mycelia of *Schizophyllum commune* and a processed product of mycelia of *Cortinellus shiitake*.

21. The composition for inducing the production of IL-12 to be administered to a patient suffering from primary cancer according to claim 20, wherein the processed product of mycelia of *Schizophyllum commune* is SCP, the polysaccharide derived from cultured filtrate of mycelia of *Schizophyllum commune* is SPG and a processed product of mycelia of *Cortinellus shiitake* is AHCC and LEM.

22. A composition for inducing the production of IL-12 in which a processed product of mycelia having an ability of inducing the production of IL-12 in a patient suffering from progressive or terminal cancer and a processed product of mycelia having an ability of inducing the production of IL-12 in a patient suffering from primary cancer are compounded.

23. The composition for inducing the production of IL-12 according to claim 22, wherein at least three selected from a processed product of mycelia of *Schizophyllum commune* or a polysaccharide derived from cultured filtrate of mycelia of *Schizophyllum commune,* a processed product of mycelia of *Cortinellus shiitake*, a processed product of mycelia of *Fomos glaucotus* and a processed product of mycelia of *Coriolus versicolor* are compounded as active ingredients.

24. The composition for inducing the production of IL-12 according to claim 23, wherein the processed product of mycelia of *Schizophyllum commune* is SCP, the polysaccharide derived from cultured filtrate of mycelia of *Schizophyllum commune* is SPG, the processed product of mycelia of *Cortinellus shiitake* is AHCC and LEM, the processed product of mycelia of *Fomos glaucotus* is SM and MAK and the processed product of mycelia of *Coriolus versicolor* is PSK (Krestin®).

25. A therapeutic agent for cancer containing any of the compositions mentioned in claims 5 to 24, **characterized in that**, the agent is in a form of being administered *per os*.

26. A supplementary food preparation for health by oral administration containing any of the compositions mentioned in claims 5 to 24.

27. A commercial medium carrying any of the contents mentioned in claims 1 to 26.

28. A commercial method utilizing any of the contents mentioned in claims 1 to 26.

29. In a method for testing the activation of NKT cells, a method for testing the activation of NKT cells which is **characterized in that** the cell surface antigens CD161 and CD3 are measured.

30. In a system where immunotherapy of cancer is carried out under tie-up and cooperation of an organization for testing the immune function, an organization for evaluating the immune function and an organization for therapy and/or formulation (a medical organization), a novel cancer immunotherapy system comprising
(1) when blood of a patient suffering from cancer is collected at the medical organization and sent to the organization for testing the immune function,
(2) measurement of at least IL-12, NKT activity and IFNγ is carried out at the said organization for testing,
(3) result of the measurement is sent to the tied-up organization for evaluating the immune function and an evaluation is carried out in the said organization on the basis of the result of the examination,
(4) whereby selection of the adaptability with the formulation of claims 1 to 26 is carried out,
(5) the said information is transmitted to the organization for therapy and/or formulation and
(6) a cancer immunotherapy is carried out on the basis of the said transmition.

31. A method for the cancer immunotherapy by any of the formulations of claims 1 to 26.
